(19) **Europäisches Patentamt**
**European Patent Office**
**Office européen des brevets**

(11) **EP 3 521 444 A1**

(12) **EUROPEAN PATENT APPLICATION**
published in accordance with Art. 153(4) EPC

(43) Date of publication:
**07.08.2019 Bulletin 2019/32**

(21) Application number: **17855596.7**

(22) Date of filing: **04.09.2017**

(51) Int Cl.:
*C12Q 1/68* (2018.01)          *C12N 15/09* (2006.01)
*C12Q 1/6844* (2018.01)

(86) International application number:
**PCT/JP2017/031764**

(87) International publication number:
**WO 2018/061638 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2016  JP 2016193958**

(71) Applicant: FUJIFILM Corporation
**Tokyo 106-8620 (JP)**

(72) Inventors:
- **INOUE, Yuki**
  **Ashigara-kami-gun**
  **Kanagawa 258-8538 (JP)**
- **TSUJIMOTO, Takayuki**
  **Ashigara-kami-gun**
  **Kanagawa 258-8538 (JP)**
- **NAKATANI, Toshiyuki**
  **Ashigara-kami-gun**
  **Kanagawa 258-8538 (JP)**

(74) Representative: **Klunker IP**
**Patentanwälte PartG mbB**
**Destouchesstraße 68**
**80796 München (DE)**

(54) **METHOD FOR DETERMINING ORIGIN OF HUMAN GENOMIC DNA OF 100 pg OR LESS THEREFROM, METHOD FOR PERSONAL IDENTIFICATION, AND METHOD FOR ANALYZING DEGREE OF ENGRAFTMENT OF HEMATOPOIETIC STEM CELL**

(57)     Provided are a method for discriminating an origin of human genomic DNA of 100 pg or less by uniformly amplifying human genomic DNA of 100 pg or less, a method for identifying an individual, and a method for analyzing a level of engraftment of hematopoietic stem cells.

FIG. 1

EP 3 521 444 A1

SEQUENCE LISTING

<110> FUJI FILM CORP.

<120> Method for designing primers used in multiplex PCR

<130> W-6026PCT

<150> JP2016-193958
<151> 2016-09-30

<160> 62

<170> PatentIn version 3.5

<210> 1
<211> 37
<212> DNA
<213> Artificial Sequence

<220>
<223> 2001-F

<400> 1
cgctcttccg atctctggtt cgatgcggac cttctgg                37

<210> 2
<211> 37
<212> DNA
<213> Artificial Sequence

<220>
<223> 2001-R

<400> 2
cgctcttccg atctgactct cccacatccg gctatgg                37

<210> 3
<211> 37
<212> DNA
<213> Artificial Sequence

<220>
<223> 2002-F

<400> 3
cgctcttccg atctctgttt ccccgaccat aagcttg                37

<210> 4
<211> 37
<212> DNA
<213> Artificial Sequence

<220>
<223> 2002-R

<400> 4
cgctcttccg atctgacata cagggctgag agattgg                37

```
<210>  5
<211>  37
<212>  DNA
<213>  Artificial Sequence

<220>
<223>  2003-F

<400>  5
cgctcttccg atctctgtga taaggtccga actttgg                          37


<210>  6
<211>  37
<212>  DNA
<213>  Artificial Sequence

<220>
<223>  2003-R

<400>  6
cgctcttccg atctgacgcg actgcaagag attcgtg                          37


<210>  7
<211>  37
<212>  DNA
<213>  Artificial Sequence

<220>
<223>  2004-F

<400>  7
cgctcttccg atctctgatt tgctgctgac cagggtg                          37


<210>  8
<211>  37
<212>  DNA
<213>  Artificial Sequence

<220>
<223>  2004-R

<400>  8
cgctcttccg atctgacagg tacagcttcc catctgg                          37


<210>  9
<211>  37
<212>  DNA
<213>  Artificial Sequence

<220>
<223>  2005-F

<400>  9
cgctcttccg atctctgccg tgtgtgagat tctcgtg                          37


<210>  10
<211>  37
<212>  DNA
```

<213> Artificial Sequence

<220>
<223> 2005-R

<400> 10
cgctcttccg atctgacact gctcagggtc ctctgtg                                37


<210> 11
<211> 37
<212> DNA
<213> Artificial Sequence

<220>
<223> 2078-F

<400> 11
cgctcttccg atctctgagg ttctgctcgt tggcttg                                37


<210> 12
<211> 37
<212> DNA
<213> Artificial Sequence

<220>
<223> 2078-R

<400> 12
cgctcttccg atctgaccca agaaggacac ggattgg                                37


<210> 13
<211> 37
<212> DNA
<213> Artificial Sequence

<220>
<223> 2079-F

<400> 13
cgctcttccg atctctgagc ttcccgggaa attagtg                                37


<210> 14
<211> 37
<212> DNA
<213> Artificial Sequence

<220>
<223> 2079-R

<400> 14
cgctcttccg atctgacgaa ttttaatcgc ccctgtg                                37


<210> 15
<211> 37
<212> DNA
<213> Artificial Sequence

<220>

<223>  2080-F

<400>  15
cgctcttccg atctctggga ctgcttagat gccgtgg                                    37

<210>  16
<211>  37
<212>  DNA
<213>  Artificial Sequence

<220>
<223>  2080-R

<400>  16
cgctcttccg atctgacagt caaaagcatg tcagtgg                                    37

<210>  17
<211>  37
<212>  DNA
<213>  Artificial Sequence

<220>
<223>  2081-F

<400>  17
cgctcttccg atctctgctc tgtggagtcc gtgatgg                                    37

<210>  18
<211>  37
<212>  DNA
<213>  Artificial Sequence

<220>
<223>  2081-R

<400>  18
cgctcttccg atctgacatg cagtcacagt ggtatgg                                    37

<210>  19
<211>  37
<212>  DNA
<213>  Artificial Sequence

<220>
<223>  2082-F

<400>  19
cgctcttccg atctctgggt aatgctgcaa gctctgg                                    37

<210>  20
<211>  37
<212>  DNA
<213>  Artificial Sequence

<220>
<223>  2082-R

<400>  20

cgctcttccg atctgaccct aggggatcaa gatgtgg                          37


<210>  21
<211>  37
<212>  DNA
<213>  Artificial Sequence

<220>
<223>  2128-F

<400>  21
cgctcttccg atctctggca agagtctggc ttttttg                          37


<210>  22
<211>  37
<212>  DNA
<213>  Artificial Sequence

<220>
<223>  2128-R

<400>  22
cgctcttccg atctgacggg atcaggtact gccgttg                          37


<210>  23
<211>  37
<212>  DNA
<213>  Artificial Sequence

<220>
<223>  2129-F

<400>  23
cgctcttccg atctctgcaa ggtgctacat gtgctgg                          37


<210>  24
<211>  37
<212>  DNA
<213>  Artificial Sequence

<220>
<223>  2129-R

<400>  24
cgctcttccg atctgacgct ttgcagggga atgtttg                          37


<210>  25
<211>  37
<212>  DNA
<213>  Artificial Sequence

<220>
<223>  2130-F

<400>  25
cgctcttccg atctctggag cagcgtacca ttgggtg                          37

```
<210>  26
<211>  37
<212>  DNA
<213>  Artificial Sequence

<220>
<223>  2130-R

<400>  26
cgctcttccg atctgaccag tgttctcgct catgtgg                                37


<210>  27
<211>  37
<212>  DNA
<213>  Artificial Sequence

<220>
<223>  2131-F

<400>  27
cgctcttccg atctctgtgt ctcccccttt ttagttg                                37


<210>  28
<211>  37
<212>  DNA
<213>  Artificial Sequence

<220>
<223>  2131-R

<400>  28
cgctcttccg atctgacttt acctggcttt ggagttg                                37


<210>  29
<211>  37
<212>  DNA
<213>  Artificial Sequence

<220>
<223>  2132-F

<400>  29
cgctcttccg atctctggtc agcaagttgg ctactgg                                37


<210>  30
<211>  37
<212>  DNA
<213>  Artificial Sequence

<220>
<223>  2132-R

<400>  30
cgctcttccg atctgacagc cttaggctcc catggtg                                37


<210>  31
<211>  37
<212>  DNA
```

```
<213>  Artificial Sequence

<220>
<223>  2169-F

<400>  31
cgctcttccg atctctgaga ctcactccac gtgtgtg                                    37


<210>  32
<211>  37
<212>  DNA
<213>  Artificial Sequence

<220>
<223>  2169-R

<400>  32
cgctcttccg atctgacaga acccagtggt gaatttg                                    37


<210>  33
<211>  37
<212>  DNA
<213>  Artificial Sequence

<220>
<223>  2170-F

<400>  33
cgctcttccg atctctgctt ccccttctgt gggtgtg                                    37


<210>  34
<211>  37
<212>  DNA
<213>  Artificial Sequence

<220>
<223>  2170-R

<400>  34
cgctcttccg atctgacagg ataaaacaat gggttgg                                    37


<210>  35
<211>  37
<212>  DNA
<213>  Artificial Sequence

<220>
<223>  2171-F

<400>  35
cgctcttccg atctctgctg ttgccgtctc ttcatgg                                    37


<210>  36
<211>  37
<212>  DNA
<213>  Artificial Sequence

<220>
```

<223> 2171-R

<400> 36
cgctcttccg atctgacacc tctggaggaa gttgttg                                    37

<210> 37
<211> 37
<212> DNA
<213> Artificial Sequence

<220>
<223> 2173-F

<400> 37
cgctcttccg atctctggaa ctccttgtgg cggcttg                                    37

<210> 38
<211> 37
<212> DNA
<213> Artificial Sequence

<220>
<223> 2173-R

<400> 38
cgctcttccg atctgaccct gcaagaaggt cttatgg                                    37

<210> 39
<211> 37
<212> DNA
<213> Artificial Sequence

<220>
<223> 2175-F

<400> 39
cgctcttccg atctctgctc catggcttgg atcttgg                                    37

<210> 40
<211> 37
<212> DNA
<213> Artificial Sequence

<220>
<223> 2175-R

<400> 40
cgctcttccg atctgacagc gcctggacag ctatgtg                                    37

<210> 41
<211> 37
<212> DNA
<213> Artificial Sequence

<220>
<223> 2176-F

<400> 41

cgctcttccg atctctgtac gccaggtgtc tcgcttg                      37


<210>  42
<211>  37
<212>  DNA
<213>  Artificial Sequence

<220>
<223>  2176-R

<400>  42
cgctcttccg atctgactcc agataaaggc ggctttg                      37


<210>  43
<211>  69
<212>  DNA
<213>  Artificial Sequence

<220>
<223>  D501

<400>  43
aatgatacgg cgaccaccga gatctacact atagccttct ttccctacac gacgctcttc    60

cgatctctg                                                     69


<210>  44
<211>  69
<212>  DNA
<213>  Artificial Sequence

<220>
<223>  D502

<400>  44
aatgatacgg cgaccaccga gatctacaca tagaggctct ttccctacac gacgctcttc    60

cgatctctg                                                     69


<210>  45
<211>  69
<212>  DNA
<213>  Artificial Sequence

<220>
<223>  D503

<400>  45
aatgatacgg cgaccaccga gatctacacc ctatccttct ttccctacac gacgctcttc    60

cgatctctg                                                     69


<210>  46
<211>  69
<212>  DNA
<213>  Artificial Sequence

<220>

<223> D504

<400> 46
aatgatacgg cgaccaccga gatctacacg gctctgatct ttccctacac gacgctcttc    60

cgatctctg    69


<210> 47
<211> 69
<212> DNA
<213> Artificial Sequence

<220>
<223> D505

<400> 47
aatgatacgg cgaccaccga gatctacaca ggcgaagtct ttccctacac gacgctcttc    60

cgatctctg    69


<210> 48
<211> 69
<212> DNA
<213> Artificial Sequence

<220>
<223> D506

<400> 48
aatgatacgg cgaccaccga gatctacact aatcttatct ttccctacac gacgctcttc    60

cgatctctg    69


<210> 49
<211> 69
<212> DNA
<213> Artificial Sequence

<220>
<223> D507

<400> 49
aatgatacgg cgaccaccga gatctacacc aggacgttct ttccctacac gacgctcttc    60

cgatctctg    69


<210> 50
<211> 69
<212> DNA
<213> Artificial Sequence

<220>
<223> D508

<400> 50
aatgatacgg cgaccaccga gatctacacg tactgactct ttccctacac gacgctcttc    60

cgatctctg    69

<210> 51
<211> 69
<212> DNA
<213> Artificial Sequence

<220>
<223> D701

<400> 51
caagcagaag acggcatacg agatcgagta atgtgactgg agttcagacg tgtgctcttc     60

cgatctgac                                                            69


<210> 52
<211> 69
<212> DNA
<213> Artificial Sequence

<220>
<223> D702

<400> 52
caagcagaag acggcatacg agattctccg gagtgactgg agttcagacg tgtgctcttc     60

cgatctgac                                                            69


<210> 53
<211> 69
<212> DNA
<213> Artificial Sequence

<220>
<223> D703

<400> 53
caagcagaag acggcatacg agataatgag cggtgactgg agttcagacg tgtgctcttc     60

cgatctgac                                                            69


<210> 54
<211> 69
<212> DNA
<213> Artificial Sequence

<220>
<223> D704

<400> 54
caagcagaag acggcatacg agatggaatc tcgtgactgg agttcagacg tgtgctcttc     60

cgatctgac                                                            69


<210> 55
<211> 69
<212> DNA
<213> Artificial Sequence

<220>

<223> D705

<400> 55
caagcagaag acggcatacg agatttctga atgtgactgg agttcagacg tgtgctcttc    60

cgatctgac    69

<210> 56
<211> 69
<212> DNA
<213> Artificial Sequence

<220>
<223> D706

<400> 56
caagcagaag acggcatacg agatacgaat tcgtgactgg agttcagacg tgtgctcttc    60

cgatctgac    69

<210> 57
<211> 69
<212> DNA
<213> Artificial Sequence

<220>
<223> D707

<400> 57
caagcagaag acggcatacg agatagcttc aggtgactgg agttcagacg tgtgctcttc    60

cgatctgac    69

<210> 58
<211> 69
<212> DNA
<213> Artificial Sequence

<220>
<223> D708

<400> 58
caagcagaag acggcatacg agatgcgcat tagtgactgg agttcagacg tgtgctcttc    60

cgatctgac    69

<210> 59
<211> 69
<212> DNA
<213> Artificial Sequence

<220>
<223> D709

<400> 59
caagcagaag acggcatacg agatcatagc cggtgactgg agttcagacg tgtgctcttc    60

cgatctgac    69

```
<210>  60
<211>  69
<212>  DNA
<213>  Artificial Sequence

<220>
<223>  D710

<400>  60
caagcagaag acggcatacg agatttcgcg gagtgactgg agttcagacg tgtgctcttc        60

cgatctgac                                                                 69


<210>  61
<211>  69
<212>  DNA
<213>  Artificial Sequence

<220>
<223>  D711

<400>  61
caagcagaag acggcatacg agatgcgcga gagtgactgg agttcagacg tgtgctcttc        60

cgatctgac                                                                 69


<210>  62
<211>  69
<212>  DNA
<213>  Artificial Sequence

<220>
<223>  D712

<400>  62
caagcagaag acggcatacg agatctatcg ctgtgactgg agttcagacg tgtgctcttc        60

cgatctgac                                                                 69
```

**Description**

**BACKGROUND OF THE INVENTION**

1. Field of the Invention

The present invention relates to a method for discriminating an origin of human genomic DNA of 100 pg or less (deoxyribonucleic acid), a method for identifying an individual, and a method for analyzing a level of engraftment of hematopoietic stem cells.

2. Description of the Related Art

In order to obtain base sequence information from a trace amount of genomic DNA, it is necessary to amplify DNA, which is an analysis object, by whole genome amplification, and then amplify a desired objective region by polymerase chain reaction (PCR). However, with this method, it is not possible to obtain highly accurate base sequence information due to unevenness in amplification regions, amplification error, or the like.

In a case where an objective region is directly amplified by PCR without performing whole genome amplification, the influence of the whole genome amplification can be eliminated. However, because an amount of template DNA is extremely small, an amplification error due to excessive amplification reaction, nonspecific amplification (primer-dimer) between primers, or the like occurs, and therefore accurate base sequence information cannot be acquired in some cases.

As a means for inhibiting the formation of primer-dimers, for example, WO2008/004691A discloses that with respect to each of combinations of primers, a score indicating complementarity at 3' terminals between primers (local alignment score at the 3' terminals) is calculated, combinations of primers with low complementarity between primers are selected, and thereby reducing a possibility that primers of different targets form primer-dimers through multiplex PCR.

**SUMMARY OF THE INVENTION**

However, it is important to consider primer-dimers formed by annealing only at a primer end in multiplex PCR for an extremely small amount of template DNA; however, the means described in WO2008/004691A does not take this condition into consideration.

Accordingly, in a primer designed according to a method for designing a primer described in WO2008/004691A, it was not possible to uniformly amplify a plurality of objective regions in a case where multiplex PCR was performed on an extremely small amount of template DNA of 100 pg or less.

Therefore, in the related art, it was difficult to discriminate an origin of a trace amount of DNA of 100 pg or less, such as retained DNA or DNA extracted from a single cell, to identify individuals, and to analyze a level of engraftment of hematopoietic stem cells.

An object of the present invention is to provide a method for discriminating an origin of human genomic DNA of 100 pg or less by uniformly amplifying human genomic DNA of 100 pg or less, a method for identifying an individual, and a method for analyzing a level of engraftment of hematopoietic stem cells.

The inventors of the present invention have conducted extensive studies to solve the above-described problems, and as a result, have found that, in a method for designing a primer used in a case of performing multiplex PCR using human genomic DNA of 100 pg or less as a template, formation of primer-dimers can be suppressed, and a plurality of objective regions can be uniformly amplified in a case where primers selected in both a first stage and a second stage are employed by performing: first stage selection based on a local alignment score obtained by evaluating formability of a primer-dimer and obtaining a local alignment score through performing pairwise local alignment on a base sequence of a primer candidate under the condition that a partial sequence to be subjected to comparison includes the 3' terminal of a base sequence of a primer; and a second stage selection based on a global alignment score obtained by performing pairwise global alignment on a base sequence which has a predetermined sequence length and includes the 3' terminal of the base sequence of the primer candidate, and therefore have completed the present invention.

That is, the present invention provides the following [1] to [6].

[1] A method for discriminating an origin of human genomic DNA of 100 pg or less, comprising:

an objective region selection step of selecting at least one objective region for obtaining base sequence information, from regions on human genomic DNA;
a DNA extraction step of extracting human genomic DNA from a sample derived from a human;
a PCR amplification step of PCR amplifying the at least one objective region by using a primer set that is designed to PCR amplify the at least one objective region and using the human genomic DNA of 100 pg or less as a template from the human genomic DNA obtained in the DNA extraction step;
a DNA sequencing step of decoding a DNA base sequence of a PCR amplification product obtained in the PCR amplification step so as to obtain the base sequence information of the at least one objective region; and
an origin discrimination step of discriminating the origin of the human genomic DNA based on the base sequence information,
in which the objective region selection step and the DNA extraction step are performed in random order, and
in which the primer set that is designed to PCR amplify the at least one objective region is designed through a method for designing a primer set used for a polymerase chain reaction, the designing method including:

a target region selection step a) of selecting a target region from the at least one objective region;
a primer candidate base sequence generation step b) of generating at least one base sequence of a primer candidate for PCR amplifying the target region based on each base sequence in each of vicinity regions at both ends of the target region on the human genomic DNA;
a local alignment step c) of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences;
a first stage selection step d) of performing first stage selection of the base sequence of the primer candidate for PCR amplifying the target region based on the local alignment score;
a global alignment step e) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step;
a second stage selection step f) of performing second stage selection of the base sequence of the primer candidate for PCR amplifying the target region based on the global alignment score; and
a primer employment step g) of employing the base sequence of the primer candidate which is selected in both of the first stage selection step and the second stage selection step as the base sequence of the primer for PCR amplifying the target region,
in which both steps of the local alignment step and the first stage selection step, and both steps of the global alignment step and the second stage selection step are performed in random order or at the same time.

[2] A method for discriminating an origin of human genomic DNA of 100 pg or less, comprising:

an objective region selection step of selecting at least one objective region for obtaining base sequence information, from regions on a human genomic DNA;
a DNA extraction step of extracting human genomic DNA from a sample derived from a human;
a PCR amplification step of PCR amplifying the at least one objective region by using a primer set that is designed to PCR amplify the at least one objective region and using the human genomic DNA of 100 pg or less as a template from the human genomic DNA obtained in the DNA extraction step;
a DNA sequencing step of decoding a DNA base sequence of a PCR amplification product obtained in the PCR amplification step so as to obtain the base sequence information of the at least one objective region; and
an origin discrimination step of discriminating the origin of the human genomic DNA based on the base sequence information,
in which the objective region selection step and the DNA extraction step are performed in random order, and
in which the primer set that is designed to PCR amplify the at least one objective region is designed through a method for designing a primer set used for a polymerase chain reaction, the designing method including:

a first step of target region selection $a_1$) of selecting a first target region from the at least one objective region;
a first step of primer candidate base sequence generation $b_1$) of generating at least one base sequence of a primer candidate for PCR amplifying the first target region based on each base sequence in each of vicinity regions at both ends of the first target region on the human genomic DNA;
a first step of local alignment $c_1$) of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences;

a first step of first stage selection $d_1$) of performing first stage selection of the base sequence of the primer candidate for PCR amplifying the first target region based on the local alignment score;

a first step of global alignment $e_1$) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection;

a first step of second stage selection $f_1$) of performing second stage selection of the base sequence of the primer candidate for PCR amplifying the first target region based on the global alignment score;

a first step of primer employment $g_1$) of employing the base sequence of the primer candidate which is selected in both of the first step of first stage selection and the first step of second stage selection as a base sequence of a primer for PCR amplifying the first target region;

a second step of target region selection $a_2$) of selecting a second target region from objective regions which have not yet been selected from the at least one objective region;

a second step of primer candidate base sequence generation $b_2$) of generating at least one base sequence of a primer candidate for PCR amplifying the second target region based on each base sequence in each of vicinity regions at both ends of the second target region on the human genomic DNA;

a second step of local alignment $c_2$) of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences;

a second step of first stage selection $d_2$) of performing first stage selection of the base sequence of the primer candidate for PCR amplifying the second target region based on the local alignment score;

a second step of global alignment $e_2$) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed;

a second step of second stage selection $f_2$) of performing second stage selection of the base sequence of the primer candidate for PCR amplifying the second target region based on the global alignment score; and

a second step of primer employment $g_2$) of employing the base sequence of the primer candidate which is selected in both of the second step of first stage selection and the second step of second stage selection as a base sequence of a primer for PCR amplifying the second target region,

in which both steps of the first step of local alignment and the first step of first stage selection, and both steps of the first step of global alignment and the first step of second stage selection are performed in random order or at the same time,

in which both steps of the second step of local alignment and the second step of first stage selection, and both steps of the second step of global alignment and the second step of second stage selection are performed in random order or at the same time, and

in which in a case where the at least one objective region has three or more objective regions, and in case of employing a base sequence of a primer for PCR amplifying third and subsequent target regions, which have not yet been selected from the three or more objective regions, each step from the second step of target region selection to the second step of primer employment is repeated for the third and subsequent target regions.

[3] A method for discriminating an origin of human genomic DNA of 100 pg or less, comprising:

an objective region selection step of selecting at least one objective region for obtaining base sequence information, from regions on human genomic DNA;

a DNA extraction step of extracting human genomic DNA from a sample derived from a human;

a PCR amplification step of PCR amplifying the at least one objective region by using a primer set that is designed to PCR amplify the at least one objective region and using the human genomic DNA of 100 pg or less as a template from the human genomic DNA obtained in the DNA extraction step;

a DNA sequencing step of decoding a DNA base sequence of a PCR amplification product obtained in the PCR amplification step so as to obtain the base sequence information of the at least one objective region; and an origin discrimination step of discriminating the origin of the human genomic DNA based on the base sequence information,

in which the objective region selection step and the DNA extraction step are performed in random order, and in which the primer set that is designed to PCR amplify the at least one objective region is designed through a method for designing a primer set used for a polymerase chain reaction, the designing method including:

a target region multiple selection step a-0) of selecting a plurality of target regions from the at least one objective region;

a primer candidate base sequence multiple generation step b-0) of generating at least one base sequence of a primer candidate for PCR amplifying the plurality of target regions based on each base sequence in each of vicinity regions at both ends of the plurality of target regions on the human genomic DNA;

a first local alignment step c-1) of obtaining a local alignment score by performing pairwise local alignment on two base sequences included in each of combinations which are obtainable by selecting base sequences of two primer candidates from the base sequences of the primer candidates for PCR amplifying the first target region among the base sequences of the primer candidates generated in the primer candidate base sequence multiple generation step, under a condition that partial sequences to be compared have 3' terminal of the two base sequences;

a first first-stage selection step d-1) of performing first stage selection of the base sequence of the primer candidate for PCR amplifying the first target region based on the local alignment score;

a first global alignment step e-1) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step;

a first second-stage selection step f-1) of performing second stage selection of the base sequence of the primer candidate for PCR amplifying the first target region based on the global alignment score;

a first primer employment step g-1) of employing the base sequence of the primer candidate which is selected in both of the first first-stage selection step and the first second-stage selection step as the base sequence of the primer for PCR amplifying the first target region;

a second local alignment step c-2) of obtaining a local alignment score by performing pairwise local alignment on two base sequences included in each of combinations which are combinations obtainable by selecting base sequences of two primer candidates from the base sequences of the primer candidates for PCR amplifying the second target region among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminal of the two base sequences;

a second first-stage selection step d-2) of performing first stage selection of the base sequence of the primer candidate for PCR amplifying the second target region based on the local alignment score;

a second global alignment step e-2) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed;

a second second-stage selection step f-2) of performing second stage selection of the base sequence of the primer candidate for PCR amplifying the second target region based on the global alignment score; and a second primer employment step g-2) of employing the base sequence of the primer candidate which is selected in both of the second first-stage selection step and the second second-stage selection step as the base sequence of the primer for PCR amplifying the second target region,

in which both steps of the first local alignment step and the first first-stage selection step, and both steps of the first global alignment step and the first second-stage selection step are performed in random order or at the same time,

in which both steps of the second local alignment step and the second first-stage selection step, and both steps of the second global alignment step and the second second-stage selection step are performed in random order or at the same time, and

in which in a case where the at least one objective region has three or more objective regions, three or more target regions are selected in the target region multiple selection step, and the base sequence of the primer candidate for PCR amplifying each of the three or more target regions is generated in the primer candidate base sequence multiple generation step, and in case of employing the base sequence of the primer for PCR amplifying third and subsequent target regions, each step from the second local alignment step to the second primer employment step is repeated for the third and subsequent target regions.

[4] The method according to any one of [1] to [3], in which the objective region includes a single nucleotide polymorphism and/or a short tandem repeat.

[5] A method for identifying an individual using the method for discriminating an origin of human genomic DNA of 100 pg or less according to any one of [1] to [4].

[6] A method for analyzing a level of engraftment of hematopoietic stem cells using the method for discriminating an origin of human genomic DNA of 100 pg or less according to any one of [1] to [4].

According to the present invention, it is possible to provide a method for discriminating an origin of human genomic DNA of 100 pg or less by uniformly amplifying human genomic DNA of 100 pg or less, a method for identifying an individual, and a method for analyzing a level of engraftment of hematopoietic stem cells.

According to the present invention, DNA analysis can be performed with high accuracy even with a trace amount of DNA of 100 pg or less. Furthermore, DNA analysis can be performed with high accuracy even with a DNA amount of 50 pg or less, which is useful for discrimination of the origin of human genomic DNA, a method for identifying an individual, and analysis of a level of engraftment of hematopoietic stem cells.

## BRIEF DESCRIPTION OF THE DRAWINGS

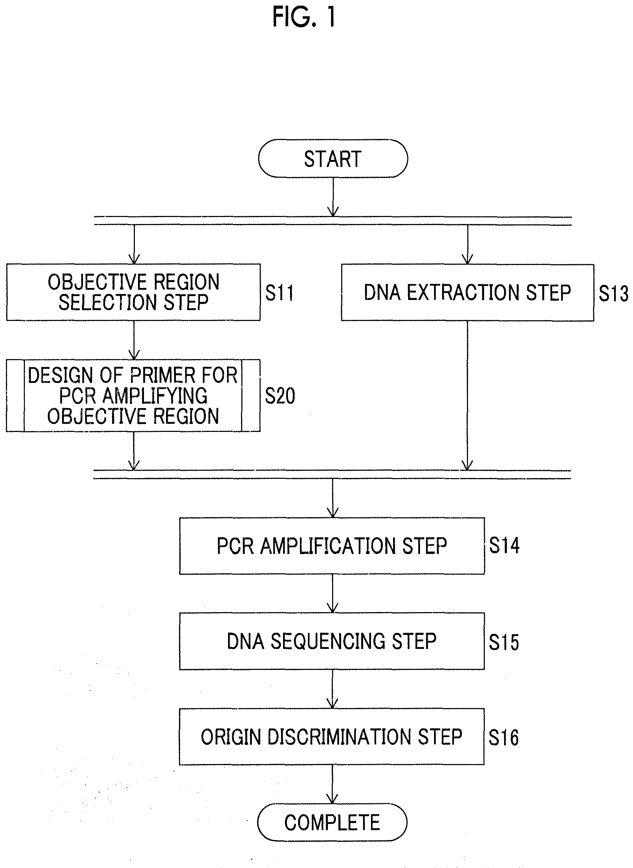

Fig. 1 is a flowchart schematically showing a method for discriminating an origin of human genomic DNA of 100 pg or less of the present invention.

Fig. 2 is a flowchart illustrating a first aspect of a method for designing a primer set used for a polymerase chain reaction for designing a primer set designed to PCR amplify an objective region to be used in the method for discriminating an origin of human genomic DNA of 100 pg or less of the present invention.

Fig. 3 is a flowchart illustrating a second aspect of a method for designing a primer set used for a polymerase chain reaction for designing a primer set designed to PCR amplify an objective region to be used in the method for discriminating an origin of human genomic DNA of 100 pg or less of the present invention.

Fig. 4 is a flowchart illustrating a second aspect of a method for designing a primer set used for a polymerase chain reaction for designing a primer set designed to PCR amplify an objective region to be used in the method for discriminating an origin of human genomic DNA of 100 pg or less of the present invention.

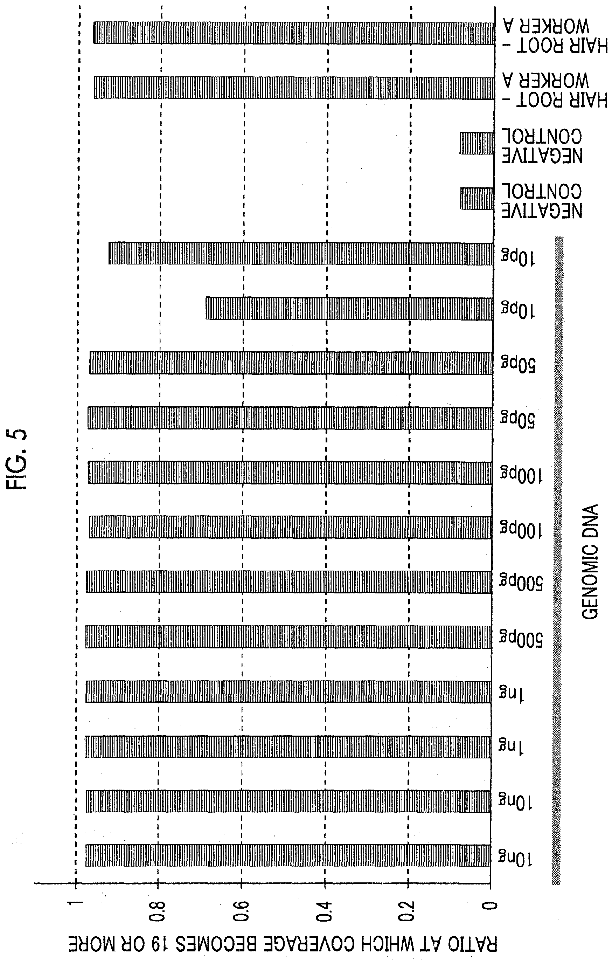

Fig. 5 is a vertical bar graph showing a relationship between an amount of template DNA (lateral axis) and a ratio of a region where a coverage in sequence reading is 19 or more (vertical axis).

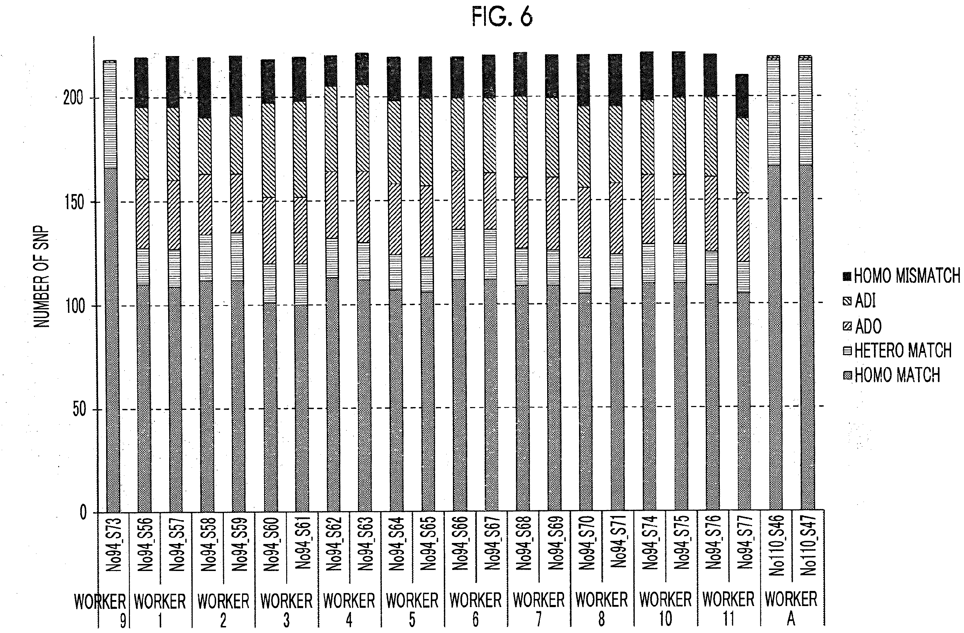

Fig. 6 is a vertical bar graph showing a relationship between workers A and 1 to 11 (lateral axis) and SNPs information [the number of homo matches, hetero mismatches, Allele Drop Out (ADO), Allele Drop In (ADI), and homo mismatches].

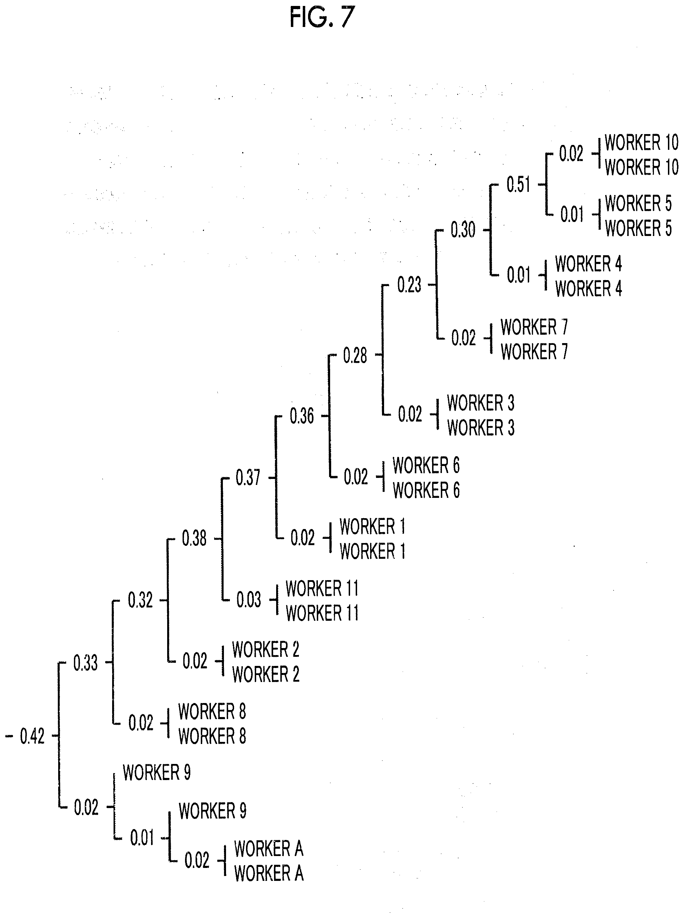

Fig. 7 is a dendrogram based on distances between workers A and 1 to 11.

## DESCRIPTION OF THE PREFERRED EMBODIMENTS

[Method for Discriminating Origin of Human Genomic DNA of 100 pg or Less]

A method for discriminating an origin of human genomic DNA of 100 pg or less of the embodiment of the present invention (hereinafter referred to as "the method for discriminating an origin of the embodiment of the present invention") comprises an objective region selection step of selecting at least one objective region for obtaining base sequence information, from regions on human genomic DNA; a DNA extraction step of extracting human genomic DNA from a sample derived from a human; a PCR amplification step of PCR amplifying the at least one objective region by using a primer set that is designed to PCR amplify the at least one objective region and using the human genomic DNA of 100 pg or less as a template from the human genomic DNA obtained in the DNA extraction step; a DNA sequencing step of decoding a DNA base sequence of a PCR amplification product obtained in the PCR amplification step so as to obtain the base sequence information of the at least one objective region; and an origin discrimination step of discriminating the origin of the human genomic DNA based on the base sequence information.

The objective region selection step and the DNA extraction step are performed in random order.

The primer set used for amplifying the objective region by the polymerase chain reaction in the PCR amplification step is designed by a method for designing a primer set described in "[Method for Designing Primer Set Used for Polymerase Chain Reaction]" to be described later.

Hereinafter, each step will be described in detail.

<Objective Region Selection Step>

In the objective region selection step, an objective region for obtaining base sequence information for discriminating the origin of human genomic DNA of 100 pg or less, which is template DNA for multiplex PCR, is selected from regions on the human genomic DNA.

(Human Genomic DNA of 100 pg or Less)

In the method for discriminating an origin of the embodiment of the present invention, the human genomic DNA used as the template DNA for multiplex PCR is not particularly limited as long as an amount thereof is 100 pg or less, but the amount is preferably 1 pg or more and 100 pg or less, more preferably 5 pg or more and 100 pg or less, and even more preferably 10 pg or more and 50 pg or less.

A lower limit value will be explained. The human genomic DNA is about 3 billion base pairs, which corresponds to about 5 pg. In the multiplex PCR to be carried out according to the present invention, approximately 110,000 base pairs can be analyzed by simultaneously amplifying about 650 sites on human genomic DNA with each of 150 to 200 base pair amplicons. This corresponds to 0.004% of human genomic DNA, and 20 fg is a theoretical lower detection limit in terms of mass. However, in practice, the amount is preferably 1 pg or more, more preferably 5 pg or more, and even more preferably 10 pg or more, in consideration of DNA polymerase, primer stability, and a state of the human genomic DNA which is an analysis object.

(Regions on Human Genomic DNA)

In the method for discriminating an origin of the embodiment of the present invention, "regions on human genomic DNA" refers to a region on genomic DNA in which a gene region having a possibility of genetic polymorphism, a single gene disease, a multifactorial disease exists. Here, the length of a region is not particularly limited, and may be one or more bases. The regions on a human genome from which an objective region is selected may exist in either a gene region or a non-gene region. The gene region contains a region of genetic polymorphism. In addition, the non-gene region includes: a non-repetitive sequence such as a pseudogene, a spacer, a response element, and a replication origin; and a repetitive sequence such as a short tandem repeat and an interspersed repetitive sequence.

Examples of genetic polymorphism include single nucleotide polymorphism (SNP), single nucleotide variant (SNV), short tandem repeat polymorphism (STRP), mutation, and insertion and/or deletion (indel).

The number of regions on human genomic DNA is not particularly limited. This is because regions on human genomic DNA are a candidate list in a case of selecting an objective region, and it is unnecessary to perform analysis for all the regions even if a large number of regions is listed.

(Objective Region)

The objective region is a region selected as a target for obtaining base sequence information from the above-described regions on human genomic DNA. The purpose of selection is not limited to detection of genetic polymorphism related to each region. In addition, the number of purposes of the selection is not limited to one, and may be two or more.

The number of regions on human genomic DNA to be selected as objective regions is at least 1 region and is not particularly limited. The number thereof is preferably greater than or equal to 3 regions, more preferably greater than or equal to 5 regions, and even more preferably greater than or equal to 10 regions.

<DNA Extraction Step>

In the DNA extraction step, human genomic DNA is extracted from a sample derived from a human.

The sample derived from a human is not particularly limited as long as the sample contains human genomic DNA, and examples therefore include cells, hair, blood, blood stains, and the like. Human genomic DNA is not limited to DNA present in the cells, and may be DNA which escaped outside the cells due to destruction of the cells or the like.

(DNA Extraction from Cells)

A method for extracting DNA from cells is carried out by heating in the presence of protease and surfactant. By this treatment, proteins and lipid components on a cell membrane are lysed, and intracellular genomic DNA can be extracted outside the cell.

It is preferable that cells to be subjected to DNA extraction are isolated. In a case of isolating the cells, cell isolation methods such as immunostaining, flow cytometry, and image discrimination which are known in the related art can be used.

<PCR Amplification Step>

In the PCR amplification step, the objective region is amplified through the polymerase chain reaction using the human genomic DNA of 100 pg or less as a template from the human genomic DNA obtained in the DNA extraction step.

The method for designing a primer set used for a polymerase chain reaction will be described in detail in "[Method for Designing Primer Set Used for Polymerase Chain Reaction]."

(Polymerase Chain Reaction)

In the polymerase chain reaction, template DNA is repeatedly replicated using DNA polymerase. The replication is started using polymerase by adding a short DNA primer hybridizing to the template DNA in a starting portion and an ending portion of a DNA base sequence to be amplified. Two chains of template double-stranded DNA are dissociated and are individually replicated every time the replication is repeated.

In multiplex PCR, it is possible to use heat-resistant DNA polymerase and a reaction buffer which are generally used in PCR. However, in some cases, each primer pair has a different temperature annealing to template DNA, and therefore, it is necessary to examine reaction conditions. For this reason, it is preferable to use heat-resistant DNA polymerase and reaction buffer which are optimized for multiplex PCR. In the present invention, it is more preferable to cause a reaction using MULTIPLEX PCR ASSAY KIT (manufactured by TAKARA BIO INC.).

The details of the method for designing a primer set will be described below. Therefore, the outline will be described herein.

Regarding the number of objective regions, target regions in necessary regions are selected from DNA sequence regions specific to target chromosomes in accordance with an examination, base sequences of primer candidates for amplifying each of the target regions are generated, and a primer candidate having low complementarity between primer base sequences is selected. Accordingly, the amplification properties of an objective region are significantly improved even with respect to a trace amount of genomic DNA such as 100 pg or less.

Although next generation sequencer technology is rapidly evolving, a very complicated process is required for preparing a sample to be used for sequence analysis. In a case of the most widely applied genome analysis, pretreatment of a sample requires processes such as (1) DNA fragmentation, (2) DNA size selection, (3) smoothing processing of DNA terminals, (4) addition of an adaptor sequence to DNA terminals, (5) Purification of DNA, and (6) amplification of DNA, after extracting nucleic acids from the sample.

The complicated sample adjustment step requires time and labor, and it is necessary to check whether the step has been appropriately performed. In addition, bias is caused in each step. Therefore, it is necessary to reduce this bias in a case of using a sample as a diagnostic tool in a medical field in which particularly high precision and accuracy of a result is required.

In order to solve these problems, in the present invention, it is possible to perform more uniform amplification of a selected objective region by performing multiplex PCR using a primer designed through a specific method to be described below. Furthermore, it is possible to more effectively collect amplification products by purifying a PCR product using magnetic beads. Contaminants such as surplus primers, deoxynucleotides (dNTPs), and enzymes remain in a PCR reaction solution. Therefore, in some cases, the remaining contaminants become an obstacle in a case of obtaining highly accurate sequence data. However, it is possible to perform sufficient purification while significantly suppressing loss of a PCR amplification product by purifying the PCR amplification product using magnetic beads. In this case, it is preferable to use a method for reliably detecting information derived from cells by uniformly amplifying various gene regions accurately through only a simple sample adjustment step such as (1) amplification of DNA and (2) purification of DNA even from an extremely small amount of DNA of 100 g or less.

Quantification of the amount of DNA amplified can be performed, for example, using NANODROP (manufactured by Thermo Fisher Scientific) which is an ultra-trace spectrophotometer for measuring the absorbance at a wavelength of 260 nm, Agilent 2100 BIOANALYZER (manufactured by Agilent Technologies) in which a laser fluorescence detection method is used, Quantus FLUOROMETER (manufactured by Promega Corporation) for quantitatively determining double-stranded DNA through a fluorescence method, or the like.

<PCR Amplification Product Purification Step>

Enzymes, nucleotides, salts, and other impurities coexist in a reaction liquid containing a PCR amplification product, and therefore are preferably removed. A method in which phenol, chloroform, and ethanol are used, a method for selectively adsorbing nucleic acids on a silica carrier such as a silica membrane filter in the presence of chaotropic salts, a method in which a phenomenon that nucleic acids are selectively bonded to magnetic beads modified with carboxyl groups in the presence of polyethylene glycol (PEG) is used, and the like are known as examples of the method for purifying nucleic acids. As the preferred embodiment of the present invention, it is possible to select a method for purifying a multiplex PCR amplification product in which magnetic beads are used.

(Magnetic Bead Purification Step)

It is possible to efficiently analyze only an objective region by purifying a PCR amplification product using magnetic beads which are paramagnetic microbeads. In the purification method in which magnetic beads are used, it is possible to efficiently remove contaminants such as enzymes, dNTPs, PCR primers, primer-dimers, and salts by reversibly bonding nucleic acids to the surfaces of particles of the magnetic beads, adsorbing the magnetic beads with a magnet, and separating an amplified DNA fragment and liquid from each other. The method in which magnetic beads are used has less sample loss and higher efficiency of removing contaminants compared to other purification methods. As a result, it is possible to efficiently analyze only an objective region in the DNA sequence step. The magnetic beads are commercially available and it is possible to use, for example, AMPure XP KIT (manufactured by BECKMAN COULTER), NucleoMag (manufactured by TAKARA BIO INC.), or EpiNext DNA Purification HT System (manufactured by Epigentek Group Inc.). Among them, it is preferable to perform purification using AMPure XP (manufactured by BECKMAN COULTER).

<DNA Sequencing Step>

A DNA base sequence of the PCR amplification product obtained in the PCR amplification step is decoded so as to obtain the base sequence information of the objective region.
It is desirable to use a next generation sequencer, particularly Miseq (manufactured by Illumina, Inc.) for analyzing a sequence of a PCR amplification product. In a case of sequencing a plurality of multiplex PCR products using the next generation sequencer "Miseq," it is necessary to add P5 and P7 sequences, which are used for hybridizing to a sample identification sequence (index sequence) formed of 6 to 8 bases, and an oligonucleotide sequence on the top of a Miseq flow cell, to each of the multiplex PCR products. By adding these sequences thereto, it is possible to measure up to 96 types of multiplex PCR products at a time. It is possible to use an adapter ligation method or a PCR method as the method for adding an index sequence and P5 and P7 sequences to both terminals of the multiplex PCR products.
In addition, in a case of mixing a plurality of multiplex PCR products and measuring the plurality of multiplex PCR products using Miseq, it is desirable to quantitatively determine each PCR product accurately. It is also possible to use Agilent 2100 BIOANALYZER (manufactured by Agilent Technologies), or Quantus FLUOROMETER (manufactured by Promega KK.) as the method for quantitatively determining PCR products. However, a method for measuring the multiplex PCR products through a quantitative PCR method is more preferable. It is preferable to perform quantitative determination as the quantitative method in the present invention using KAPA Library Quantification KIT manufactured by NIPPON Genetics Co, Ltd.
As the method for analyzing sequence data obtained using Miseq, it is preferable to map the sequence data in a well-known human genome sequence using Burrows-Wheeler Aligner (BWA: Li, H., et al., "Fast and accurate short read alignment with Burrows-Wheeler transform," Bioinformatics, 2009, Vol. 25, No. 14, PP. 1754-1760; and Li, H., et al., "Fast and accurate long-read alignment with Burrows-Wheeler transform," Bioinformatics, 2010, Vol. 26, No. 5, PP. 589-595). As means for analyzing a genetic abnormality, it is preferable to analyze genetic mutation or quantitative determination of the number of chromosomes, using SAMtools (Li, Heng, et al., "The Sequence Alignment / Map format and SAMtools," Bioinformatics, 2009, Vol. 25, No. 16, PP. 2078-2079; SAM is derived from "Sequence Alignment / Map") and/or BEDtools (Quinlan, A. R., et al., "BEDtools: a flexible suite of utilities for comparing genomic features," Bioinformatics, 2010, Vol. 26, No. 6, PP. 841-842).

(Determination of Number of Times of Sequence Reading)

In the DNA sequencing step, it is desirable to measure the number of times of sequence reading.
For example, regarding DNA fragments which are obtained by performing PCR amplification of a target region, the amplification amount (number of times of sequence reading) of amplification product having a sequence of a region of 140 bp to 180 bp which has been previously determined can be obtained using a sequencer. Regarding a cell which has been identified as cells of an individual to be suspected in criminal investigations, the amplification amount (number of times of sequence reading) of amplification product having a sequence of a region of 140 bp to 180 bp which has been previously determined is obtained as a standard (or a reference) using the sequencer.

<Origin Discrimination Step>

The origin of the human genomic DNA is discriminated based on the base sequence information.

The origin of DNA can be discriminated by, for example, a degree of similarity of the obtained base sequence information. In principle, the same base sequence information can be obtained from DNA derived from the same person. However, in practice, completely identical base sequence information may not be obtained in some cases due to ADO (Allele Drop Out), partial DNA deficiency, experimental error, or the like.

In general, a method for detecting genetic polymorphism existing in an allele is performed as a method for identifying individual differences in a gene sequence. For example, it is also possible to use STR as a type of genetic polymorphism for father-child discrimination. In addition, as a method for identifying individual differences, it is also possible to use SNPs of which a single base in a genome base sequence is mutated and which is observed at a frequency of 1% or more. In the present invention, whether or not the cells isolated from the remains have the same STR or SNPs as those of the suspect is confirmed by determining a state of DNA sequence status by using a next generation sequencer.

[Method for Designing Primer Set Used for Polymerase Chain Reaction]

The method for designing a primer set used for a polymerase chain reaction, which is carried out in a method for obtaining base sequence information of a single cell derived from a vertebrate of the present invention, will be described.

<First Aspect of Method for Designing Primer Set Used for Polymerase Chain Reaction>

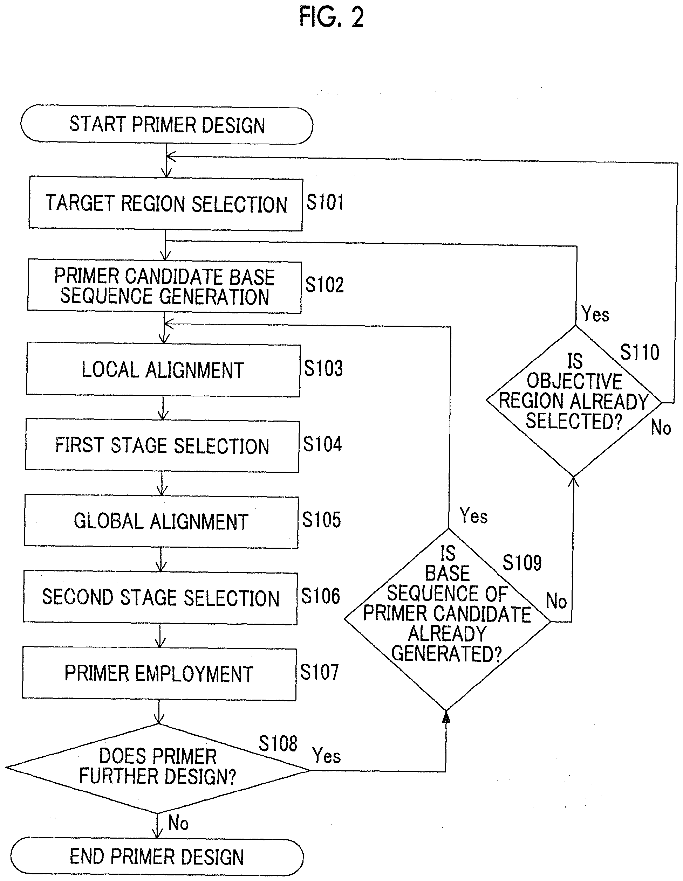

In the present invention, the first aspect of the method for designing a primer set used for a polymerase chain reaction includes the following steps. In the following description, Fig. 2 is appropriately referred to.

(a) Target region selection step of selecting a target region from at least one objective region (S101 in Fig. 2).
(b) Primer candidate base sequence generation step of generating at least one base sequence of a primer candidate for PCR amplifying the target region based on each base sequence in each of vicinity regions at both ends of the target region on the vertebrate genomic DNA (S102 in Fig. 2).
(c) Local alignment step of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences (S103 in Fig. 2).
(d) First stage selection step of performing first stage selection of the base sequence of the primer candidate for PCR amplifying the target region based on the local alignment score (S104 in Fig. 2).
(e) Global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step (S105 in Fig. 2).
(f) Second stage selection step of performing second stage selection of the base sequence of the primer candidate for PCR amplifying the target region based on the global alignment score (S106 in Fig. 2).
(g) Primer employment step of employing the base sequence of the primer candidate which is selected in both of the first stage selection step and the second stage selection step as the base sequence of the primer for PCR amplifying the target region (S107 in Fig. 2).

However, among the above steps (a) to (g), both of the above steps (c) and (d) and both of the above steps (e) and (f) are performed in random order or at the same time. That is, the steps (e) and (f) may be performed after the step (c) and the step (d) are performed, or the steps (c) and (d) may be performed after the step (e) and the step (f) are performed, or the steps (c) and (d), and the steps (e) and (f) may be performed in parallel.

In a case where the steps (c) and (d) are carried out after carrying out the steps (e) and (f), the steps (e) and (c) are preferably the following steps (e') and (c'), respectively.

(e') Global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the primer candidate base sequence generation step.

(c') Local alignment step of obtaining a local alignment score by performing pairwise local alignment on two base sequences included in each of combinations which are obtainable by selecting base sequences of two primer candidates from the base sequences of the primer candidates selected in the second stage selection step, under a condition that partial sequences to be compared have 3' terminal of the two base sequences.

In addition, in a case where the step (c) and the step (d) are carried out in parallel with the step (e) and the step (f), the step (e) is preferably the following step (e').

(e') Global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the primer candidate base sequence generation step.

Furthermore, in a case of designing a primer and in a case where a base sequence of a primer candidate has been generated, a primer is designed (S108, S109, and S110 in Fig. 2) from the b) primer candidate base sequence generation step (S102 in Fig. 2) or the a) target region selection step (S101 in Fig. 2) in a case where the base sequence of the primer candidate has not been generated from the c) local alignment step (S103 in Fig. 2).

<Second Aspect of Method for Designing Primer Set Used for Polymerase Chain Reaction>

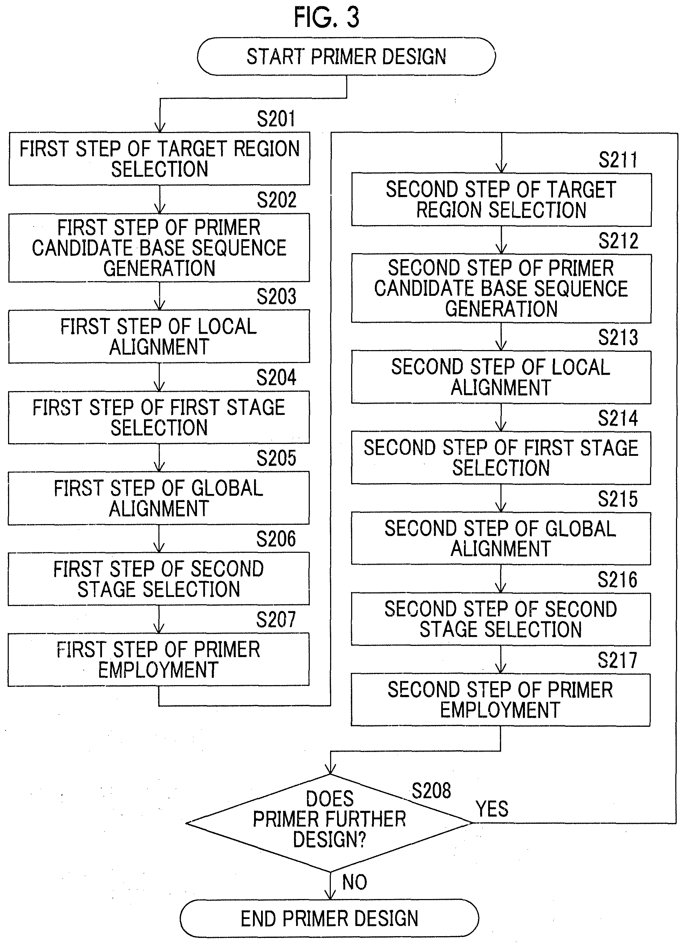

In the present invention, the second aspect, which is one of derivation forms of the method for designing a primer set used for a polymerase chain reaction in the case where at least one objective region contains two or more objective regions, includes the following steps. In the following description, Fig. 3 is appropriately referred to.

$(a_1)$ First step of target region selection of selecting a first target region from the at least one objective region (S201 in Fig. 3).

$(b_1)$ First step of primer candidate base sequence generation of generating at least one base sequence of a primer candidate for PCR amplifying the first target region based on each base sequence in each of vicinity regions at both ends of the first target region on the vertebrate genomic DNA (S202 in Fig. 2).

$(c_1)$ First step of local alignment of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences (S203 in Fig. 3).

$(d_1)$ First step of first stage selection of performing first stage selection of the base sequence of the primer candidate for PCR amplifying the first target region based on the local alignment score (S204 in Fig. 3).

$(e_1)$ First step of global alignment of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection (S205 in Fig. 3).

$(f_1)$ First step of second stage selection of performing second stage selection of the base sequence of the primer candidate for PCR amplifying the first target region based on the global alignment score (S206 in Fig. 3).

$(g_1)$ First step of primer employment of employing the base sequence of the primer candidate which is selected in both of the first step of first stage selection and the first step of second stage selection as a base sequence of a primer for PCR amplifying the first target region (S207 in Fig. 3).

However, among the above steps $(a_1)$ to $(g_1)$, both of the above steps $(c_1)$ and $(d_1)$ and both of the above steps $(e_1)$ and $(f_1)$ are performed in random order or at the same time. That is, the steps $(e_1)$ and $(f_1)$ may be performed after the step $(c_1)$ and the step $(d_1)$ are performed, or the steps $(c_1)$ and $(d_1)$ may be performed after the step $(e_1)$ and the step $(f_1)$ are performed, or the steps $(c_1)$ and $(d_1)$, and the steps $(e_1)$ and $(f_1)$ may be performed in parallel.

In a case where the steps $(c_1)$ and $(d_1)$ are carried out after carrying out the steps $(e_1)$ and $(f_1)$, the steps $(e_1)$ and $(c_1)$ are preferably the following steps $(e_1')$ and $(c_1')$, respectively.

$(e_1')$ First step of global alignment of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the first step of primer candidate base sequence generation.

($c_1$') First step of local alignment of obtaining a local alignment score by performing pairwise local alignment on two base sequences included in each of combinations which are obtainable by selecting base sequences of two primer candidates from the base sequences of the primer candidates selected in the first step of second stage selection, under a condition that partial sequences to be compared have 3' terminal of the two base sequences.

In addition, in a case where the step ($c_1$) and the step ($d_1$) are carried out in parallel with the step ($e_1$) and the step ($f_1$), the step ($e_1$) is preferably the following step ($e_1$').

($e_1$') First step of global alignment of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the first step of primer candidate base sequence generation.

($a_2$) Second step of target region selection of selecting a second target region from objective regions which have not yet been selected from the at least one objective region (S211 in Fig. 3).

($b_2$) Second step of primer candidate base sequence generation of generating at least one base sequence of a primer candidate for PCR amplifying the second target region based on each base sequence in each of vicinity regions at both ends of the second target region on the vertebrate genomic DNA (S212 in Fig. 3).

($c_2$) Second step of local alignment of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences (S213 in Fig. 3).

($d_2$) Second step of first stage selection of performing first stage selection of the base sequence of the primer candidate for PCR amplifying the second target region based on the local alignment score (S214 in Fig. 3).

($e_2$) Second step of global alignment of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed (S215 in Fig. 3).

($f_2$) Second step of second stage selection of performing second stage selection of the base sequence of the primer candidate for PCR amplifying the second target region based on the global alignment score (S216 in Fig. 3).

($g_2$) Second step of primer employment of employing the base sequence of the primer candidate which is selected in both of the second step of first stage selection and the second step of second stage selection as a base sequence of a primer for PCR amplifying the second target region (S217 in Fig. 3).

However, among the above steps ($a_2$) to ($g_2$), both of the above steps ($c_2$) and ($d_2$) and both of the above steps ($e_2$) and ($f_2$) are performed in random order or at the same time. That is, the steps ($e_2$) and ($f_2$) may be performed after the step ($c_2$) and the step ($d_2$) are performed, or the steps ($c_2$) and ($d_2$) may be performed after the step ($e_2$) and the step ($f_2$) are performed, or the steps ($c_1$) and (di), and the steps ($e_1$) and ($f_1$) may be performed in parallel.

In a case where the steps ($c_2$) and ($d_2$) are carried out after carrying out the steps ($e_2$) and ($f_2$), the steps ($e_2$) and ($c_2$) are preferably the following steps ($e_2$') and ($c_2$'), respectively.

($e_2$') Second step of global alignment of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed.

($c_2$') Second step of local alignment of obtaining a local alignment score by performing pairwise local alignment on two base sequences included in each of combinations which are combinations obtainable by selecting base sequences of two primer candidates from the base sequences of the primer candidates selected in the second step of second stage selection and base sequences of a primer already employed, and combinations obtainable by selecting a base sequence of one primer candidate and a base sequence of one primer already employed, under a condition that partial sequences to be compared have 3' terminal of the two base sequences.

In addition, in a case where the step ($c_2$) and the step ($d_2$) are carried out in parallel with the step ($e_2$) and the step ($f_2$), the step ($e_2$) is preferably the following step ($e_2'$).

($e_2'$) Second step of global alignment of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed.

In the case of further designing the primer, steps from the $a_2$) second step of target region selection (S211 in Fig. 3) to the $g_2$) second step of primer employment (S217 in Fig. 3) are repeated (S208 in Fig. 3). That is, in a case where the at least one objective region has three or more objective regions, and in case of employing a base sequence of a primer for PCR amplifying third and subsequent target regions, which have not yet been selected from the three or more objective regions, each step from the ($a_2$) step to the ($g_2$) step is repeated for the third and subsequent target regions.

<Third Aspect of Method for Designing Primer Set Used for Polymerase Chain Reaction>

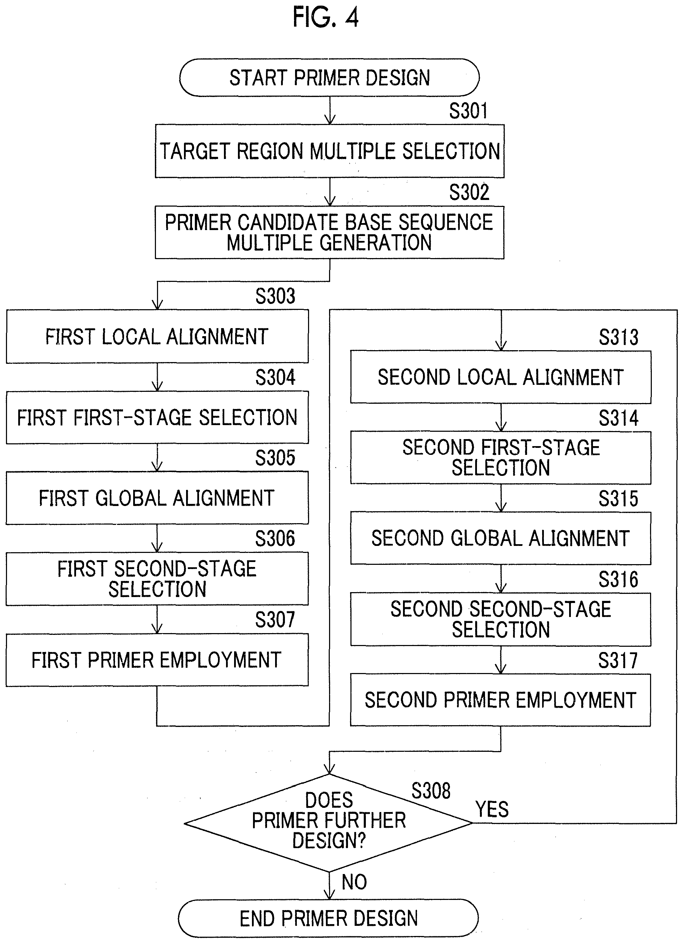

In the present invention, the third aspect, which is one of derivation forms of the method for designing a primer set used for a polymerase chain reaction in the case where at least one objective region contains two or more objective regions, includes the following steps. In the following description, Fig. 4 is appropriately referred to.

(a-0) Target region multiple selection step of selecting a plurality of target regions from the at least one objective region (S301 in Fig. 4).

(b-0) Primer candidate base sequence multiple generation step of generating at least one base sequence of a primer candidate for PCR amplifying the plurality of target regions based on each base sequence in each of vicinity regions at both ends of the plurality of target regions on the genomic DNA of the vertebrate (S302 in Fig. 4).

(c-1) First local alignment step of obtaining a local alignment score by performing pairwise local alignment on two base sequences included in each of combinations which are obtainable by selecting base sequences of two primer candidates from the base sequences of the primer candidates for PCR amplifying the first target region among the base sequences of the primer candidates generated in the primer candidate base sequence multiple generation step, under a condition that partial sequences to be compared have 3' terminal of the two base sequences (S303 in Fig. 4).

(d-1) First first-stage selection step of performing first stage selection of the base sequence of the primer candidate for PCR amplifying the first target region based on the local alignment score (S304 in Fig. 4).

(e-1) First global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step (S305 in Fig. 4).

(f-1) First second-stage selection step of performing second stage selection of the base sequence of the primer candidate for PCR amplifying the first target region based on the global alignment score (S306 in Fig. 4).

(g-1) First primer employment step of employing the base sequence of the primer candidate which is selected in both of the first first-stage selection step and the first second-stage selection step as the base sequence of the primer for PCR amplifying the first target region (S307 in Fig. 4).

However, among the above steps (c-1) to (g-1), both of the above steps (c-1) and (d-1) and both of the above steps (e-1) and (f-1) are performed in random order or at the same time. That is, the steps (e-1) and (f-1) may be performed after the step (c-1) and the step (d-1) are performed, or the steps (c-1) and (d-1) may be performed after the step (e-1) and the step (f-1) are performed, or the steps (c-1) and (d-1), and the steps (e-1) and (f-1) may be performed in parallel.

In a case where the steps (c-1) and (d-1) are carried out after carrying out the steps (e-1) and (f-1), the steps (e-1) and (c-1) are preferably the following steps (e'-1) and (c'-1), respectively.

(e'-1) First global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates for PCR amplifying the first target region among the base sequences of the primer candidates generated in the primer candidate base sequence multiple generation step.

(c'-1) First local alignment step of obtaining a local alignment score by performing pairwise local alignment on two base sequences included in each of combinations which are obtainable by selecting base sequences of two primer candidates from base sequences of primer candidates selected in the first second-stage selection step, under a condition that partial sequences to be compared have 3' terminals of the two base sequences.

In addition, in a case where the step (c-1) and the step (d-1) are carried out in parallel with the step (e-1) and the step (f-1), the step (e-1) is preferably the following step (e'-1).

(e'-1) First global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates for PCR amplifying the first target region among the base sequences of the primer candidates generated in the primer candidate base sequence multiple generation step.

(c-2) Second local alignment step of obtaining a local alignment score by performing pairwise local alignment on two base sequences included in each of combinations which are combinations obtainable by selecting base sequences of two primer candidates from base sequences of primer candidates for PCR amplifying the second target region among the base sequences of the primer candidates generated in the primer candidate base sequence multiple generation step, and base sequences of a primer already employed, and combinations obtainable by selecting a base sequence of one primer candidate and a base sequence of one primer already employed, under a condition that partial sequences to be compared have 3' terminal of the two base sequences (S313 in Fig. 4).

(d-2) Second first-stage selection step of performing first stage selection of the base sequence of the primer candidate for PCR amplifying the second target region based on the local alignment score (S314 in Fig. 4).

(e-2) Second global alignment step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed (S315 in Fig. 4).

(f-2) Second second-stage selection step of performing second stage selection of the base sequence of the primer candidate for PCR amplifying the second target region based on the global alignment score (S316 in Fig. 4).

(g-2) Second primer employment step of employing the base sequence of the primer candidate which is selected in both of the second first-stage selection step and the second second-stage selection step as the base sequence of the primer for PCR amplifying the second target region (S317 in Fig. 4).

However, among the above steps (c-2) to (g-2), both of the above steps (c-2) and (d-2) and both of the above steps (e-2) and (f-2) are performed in random order or at the same time. That is, the steps (e-2) and (f-2) may be performed after the step (c-2) and the step (d-2) are performed, or the steps (c-2) and (d-2) may be performed after the step (e-2) and the step (f-2) are performed, or the steps (c-1) and (d-1), and the steps (e-1) and (f-1) may be performed in parallel.

In a case where the steps (c-2) and (d-2) are carried out after carrying out the steps (e-2) and (f-2), the steps (e-2) and (c-2) are preferably the following steps (e'-2) and (c'-2), respectively.

(e'-2) Second global alignment step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from base sequences of primer candidates for PCR amplifying the second target region among the base sequences of the primer candidates generated in the primer candidate base sequence multiple generation step, and base sequences of a primer already employed, and combinations obtainable by selecting a base sequence of one primer candidate and a base sequence of one primer already employed.

(c'-2) Second local alignment step of obtaining a local alignment score by performing pairwise local alignment on two base sequences included in each of combinations which are combinations obtainable by selecting base sequences of two primer candidates from the base sequences of the primer candidates selected in the second second-stage selection step and base sequences of a primer already employed, and combinations obtainable by selecting a base sequence of one primer candidate and a base sequence of one primer already employed, under a condition that partial sequences to be compared have 3' terminal of the two base sequences.

In addition, in a case where the step (c-2) and the step (d-2) are carried out in parallel with the step (e-2) and the step (f-2), the step (e-2) is preferably the following step (e'-2).

(e'-2) Second global alignment step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from base sequences of primer candidates for PCR amplifying the second target region among the base sequences of the primer candidates generated in the primer candidate base sequence multiple generation step, and base sequences of a primer already employed, and combinations obtainable by selecting a base sequence of one primer candidate and a base sequence of one primer already employed.

In the case of further designing the primer, steps from the c-2) second local alignment step (S313 in Fig. 4) to the g-2) second primer employment step (S317 in Fig. 4) are repeated (S308 in Fig. 3). That is, in a case where the at least one objective region has three or more objective regions, three or more target regions are selected in the target region multiple selection step, and the base sequence of the primer candidate for PCR amplifying each of the three or more target regions is generated in the primer candidate base sequence multiple generation step, and in case of employing the base sequence of the primer for PCR amplifying third and subsequent target regions, each step from the second local alignment step to the second primer employment step is repeated for the third and subsequent target regions.

<Description of Each Step>

Hereinafter, each step included in the method for designing a primer set used for a polymerase chain reaction, which is carried out in a method for obtaining base sequence information of a single cell derived from a vertebrate of the present invention, will be described.

«Target Region Selection Step»

In the present specification, the target region selection step S101 (Fig. 2), the first step of target region selection S201 and the second step of target region selection S211 (Fig. 3), and the target region multiple selection step S301 (Fig. 4) will be collectively referred to as a "target region selection step" in some cases.

(First Aspect: Target Region Selection Step S101)

This step is shown as "target region selection" (S101) in Fig. 2.

In the first aspect, (a) target region selection step is a step of selecting a target region from an objective region.

(Second Aspect: First Step of Target Region Selection S201 and Second Step of Target Region Selection S211)

These steps are shown as "first step of target region selection" (S201) and "second step of target region selection" (S211) in Fig. 3.

In the second aspect, the ($a_1$) first step of target region selection is a step of selecting a first target region from an objective region, and the ($a_2$) second step of target region selection is a step of selecting a second target region from an objective region which has not yet been selected.

In the second aspect, objective regions are selected one by one.

(Third aspect: Target Region Multiple Selection Step S301)

This step is shown as "target region multiple selection" (S301) in Fig. 4.

In the third aspect, (a-0) target region multiple selection step is a step of selecting a plurality of target regions from an objective region.

In the third aspect, a plurality of objective regions are selected. All objective regions are preferably selected as target regions.

«Primer Candidate Base Sequence Generation Step»

In the present specification, the primer candidate base sequence generation step S102 (Fig. 2), the first step of primer candidate base sequence generation S202 and the second step of primer candidate base sequence generation S212 (Fig. 3), and the primer candidate base sequence multiple generation step S302 (Fig. 4) will be collectively referred to as a "primer candidate base sequence generation step" in some cases.

(First Aspect: Primer Candidate Base Sequence Generation Step S102)

This step is shown as "primer candidate base sequence generation" (S102) in Fig. 2.
In the first aspect, the (b) primer candidate base sequence generation step is a step of generating at least one base sequence of a primer candidate for PCR amplifying the target region based on each base sequence in each of vicinity regions at both ends of the target region on the genomic DNA.

(Second Aspect: First Step of Primer Candidate Base Sequence Generation S202 and Second Step of Primer Candidate Base Sequence Generation S212)

These steps are shown as "first step of primer candidate base sequence generation" (S202) and "second step of primer candidate base sequence generation" (S212) in Fig. 3.
In the second aspect, the ($b_1$) first step of primer candidate base sequence generation is a step of generating at least one base sequence of a primer candidate for PCR amplifying the first target region based on each base sequence in each of vicinity regions at both ends of the first target region on the genomic DNA, and the ($b_2$) second step of primer candidate base sequence generation is a step of generating at least one base sequence of a primer candidate for PCR amplifying the second target region based on each base sequence in each of vicinity regions at both ends of the second target region on the genomic DNA.
In the second aspect, with respect to one target region, generation of a base sequence of a primer candidate, selection of a primer candidate, and employment of a primer are carried out, and the same steps are repeated for the next one target region.

(Third Aspect: Primer Candidate Base Sequence Multiple Generation Step S302)

This step is shown as "primer candidate base sequence multiple generation" (S302) in Fig. 4.
In the third aspect, the (b-0) primer candidate base sequence multiple generation step is a step of generating at least one base sequence of a primer candidate for PCR amplifying the plurality of target regions based on each base sequence in each of vicinity regions at both ends of the plurality of target regions on the genomic DNA.
In the third aspect, a base sequence of a primer candidate is generated for all of the plurality of target regions, and selection and employment are repeated in subsequent steps.

(Vicinity Regions)

Each vicinity regions of the target region at both ends are collectively referred to as regions on the outside of the 5' terminal of the target region and regions on the outside of the 3' terminal of the target region. The inside of the target region is not included in the vicinity regions.
A length of the vicinity region is not particularly limited, but is preferably less than or equal to a length that can be expanded through PCR and more preferably less than or equal to the upper limit of a fragment length of DNA for which amplification is desired. A length facilitating application of concentration selection and/or sequence reading is particularly preferable. A length of the vicinity region may be appropriately changed in accordance with the type of enzyme (DNA polymerase) used for PCR. A specific length of the vicinity region is preferably about 20 to 500 bases, more preferably about 20 to 300 bases, still more preferably about 20 to 200 bases, and particularly preferably about 50 to 200 bases.

(Design Parameter of Primer)

In addition, in a case of generating a base sequence of a primer candidate, points, such as the length of a primer, the GC content (referring to a total mole percentage of guanine (G) and cytosine (C) in all nucleic acid bases), a melting temperature (which is a temperature at which 50% of double-stranded DNA is dissociated and becomes single-stranded DNA, and in which Tm is derived from a melting temperature and is referred to as "Tm value" in some cases, and the unit is "°C"), and deviation of a sequence, to be taken into consideration in a general method for designing a primer are the same.

• Length of Primer

A length of a primer (the number of nucleotides) is not particularly limited, but is preferably 15 mer to 45 mer, more preferably 20 mer to 45 mer, and still more preferably 20 mer to 30 mer. In a case where a length of a primer is within this range, it is easy to design a primer excellent in specificity and amplification efficiency. "mer" is a unit in a case where a length of polynucleotide is expressed by the number of nucleotides, and 1 mer represents one nucleotide. Therefore, for example, 15 mer represents a polynucleotide consisting of 15 nucleotides.

• GC Content of Primer

A GC content of the primer is not particularly limited, but is preferably 40 mol% to 60 mol% and more preferably 45 mol% to 55 mol%. In a case where a GC content is within this range, a problem such as a decrease in the specificity and the amplification efficiency due to a high-order structure is less likely to occur.

• Tm Value of Primer

A Tm value of the primer is not particularly limited, but is preferably within a range of 50°C to 65°C and more preferably within a range of 55°C to 65°C.
A difference in the Tm value of the primer is preferably 5°C or less and more preferably 3°C or less, in a primer pair and primer set.
Tm values can be calculated using software such as OLIGO Primer Analysis Software (manufactured by Molecular Biology Insights) or Primer 3 (http://www-genome.wi.mit.edu/ftp/distribution/software/) and the like.
In addition, the Tm value can also be obtained through calculation using the following formula from the number of A's, T's, G's, and C's (which are respectively set as nA, nT, nG, and nC) in a base sequence of a primer.

$$\text{Tm value (°C)} = 2(nA + nT) + 4(nC + nG)$$

The method for calculating the Tm value is not limited thereto and can be calculated through various well-known methods in the related art.

• Deviation of Base of Primer

The base sequence of a primer candidate is preferably set as a sequence in which there is no deviation of bases as a whole. For example, it is desirable to avoid a GC-rich sequence and a partial AT-rich sequence.
In addition, it is also desirable to avoid continuation of T and/or C (polypyrimidine) and continuation of A and/or G (polypurine).

• 3' Terminal of Primer

Furthermore, it is preferable that a 3' terminal base sequence avoids a GC-rich sequence or an AT-rich sequence. G or C is preferable for a 3' terminal base, but is not limited thereto.

«Specificity-Checking Step»

A specificity-checking step of evaluating specificity of a base sequence of a primer candidate may be performed based on sequence complementarity with respect to chromosomal DNA of a base sequence of each primer candidate which has been generated in the "primer candidate base sequence generation step."
In the specificity check, in a case where local alignment of a base sequence of chromosomal DNA and a base sequence of a primer candidate is performed and a local alignment score is less than a predetermined value, it is possible to evaluate that the complementarity of the base sequence of the primer candidate with respect to genomic DNA is low and the specificity of the base sequence of the primer candidate with respect to genomic DNA is high. Here, it is desirable to perform local alignment on also a complementary chain of chromosomal DNA. This is because chromosomal DNA is double-stranded whereas the primer is single-stranded DNA. In addition, a base sequence complementary to the base sequence of the primer candidate may be used instead of the base sequence of the primer candidate.
In addition, homology search may be performed on genomic DNA base sequence database using the base sequence of the primer candidate as a query sequence. Examples of a homology search tool include Basic Local Alignment Search Tool (BLAST) (Altschul, S. A., et al., "Basic Local Alignment Search Tool", Journal of Molecular Biology, 1990, October, Vol. 215, pp. 403-410) and FASTA (Pearson, W. R., et al., "Improved tools for biological sequence comparison", Proceedings of the National Academy of Sciences of the United States of America, National Academy of Sciences, 1988, April, Vol. 85, pp. 2444-2448). It is possible to obtain local alignment as a result of performing the homology search.

All of the scores and a threshold value of a local alignment score are not particularly limited, and can be appropriately set in accordance with the length of a base sequence of a primer candidate and/or PCR conditions, and the like. In a case of using a homology search tool, a default value of the homology search tool may be used.

For example, as the scores, it is considered that match (complementary base) = +1, mismatch (non-complementary base) = -1, and an indel (insertion and/or deletion) = -3 are employed, and the threshold value is set to be +15.

In a case where a base sequence of a primer candidate has complementarity to a base sequence at an unexpected position on chromosomal DNA but has low specificity thereto, in some cases, an artifact is amplified instead of a target region in a case where PCR is performed using a primer of the base sequence of a primer candidate. Therefore, the case where the base sequence of the primer candidate has complementarity to the base sequence at an unexpected position on genomic DNA but has low specificity thereto is excluded.

«Local Alignment Step»

In the present specification, the local alignment step S103 (Fig. 2), the first step of local alignment S203 and the second step of local alignment S213 (Fig. 3), and the first local alignment step S303 and the second local alignment step S313 (Fig. 4) will be collectively referred to as a "local alignment step" in some cases.

(First Aspect: Local Alignment Step S103)

This step is shown as "local alignment" (S103) in Fig. 2.

In the first aspect, the (c) local alignment step is a step of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences.

(Second Aspect: First Step of Local Alignment S203 and Second Step of Local Alignment S213)

These steps are shown as "first step of local alignment" (S203) and "second step of local alignment" (S213) in Fig. 3.

In the second aspect, the ($c_1$) first step of local alignment is a step of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences. The ($c_2$) second step of local alignment is a step of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences.

(Third Aspect: First Local Alignment Step S303 and Second Local Alignment Step S313)

These steps are shown as "first local alignment" (S303) and "second local alignment" (S313) in Fig. 4.

In the third aspect, the (c-1) first local alignment step is a step of obtaining a local alignment score by performing pairwise local alignment on two base sequences included in each of combinations which are obtainable by selecting base sequences of two primer candidates from the base sequences of the primer candidates for PCR amplifying the first target region which are selected from the base sequences of the primer candidates generated in the primer candidate base sequence multiple generation step, under a condition that partial sequences to be compared have 3' terminal of the two base sequences. The (c-2) second local alignment step is a step of obtaining a local alignment score by performing pairwise local alignment on two base sequences included in each of combinations which are combinations obtainable by selecting base sequences of two primer candidates from the base sequences of the primer candidates for PCR amplifying the second target region selected among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminal of the two base sequences.

(Local Alignment Method)

A combination of base sequences to be subjected to local alignment may be a combination selected while allowing overlapping, or may be a combination selected without allowing overlapping. However, in a case where formability of a primer-dimer between primers of an identical base sequence has not yet been evaluated, the combination selected while allowing overlapping is preferable.

The total number of combinations is "$_pH_2 = {}_{p+1}C_2 = (p+1)! / 2(p-1)!$" in a case where the selection is performed while allowing overlapping, and is "$_pC_2 = p(p-1) / 2$" in a case where the selection is performed without allowing overlapping, in which the total number of base sequences for performing local alignment is set to be p.

Local alignment is alignment which is performed on a partial sequence and in which it is possible to locally check a portion with high complementarity.

However, in the present invention, the local alignment is different from local alignment usually performed on a base sequence, and is designed such that partial sequences to be subjected to comparison include the 3' terminals of both base sequences by performing local alignment under the condition that the "partial sequences to be subjected to comparison include the 3' terminals of the base sequences."

Furthermore, in the present invention, an aspect is preferable in which partial sequences to be subjected to comparison include the 3' terminals of both base sequences by performing local alignment under the condition that the "partial sequences to be subjected to comparison include the 3' terminals of the base sequences," that is, the condition that "only alignments in which a partial sequence to be subjected to comparison begins at the 3' terminal of one sequence and ends at the 3' terminal of the other sequence."

Local alignment may be performed by inserting a gap. The gap means insertion and/or deletion (indel) of a base.

In addition, in the local alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, scores may be set as in Table 1. "-" in Table 1 represents a gap (insertion and/or deletion (indel)).

[Table 1]

|   | A | T | G | C | - |
|---|---|---|---|---|---|
| A | -1 | +1 | -1 | -1 | -1 |
| T | +1 | -1 | -1 | -1 | -1 |
| G | -1 | -1 | -1 | +1 | -1 |
| C | -1 | -1 | +1 | -1 | -1 |
| - | -1 | -1 | -1 | -1 |  |

"-" : gap (indel)

For example, it is considered that local alignment is performed on base sequences of SEQ ID No: 1 and SEQ ID No: 2 shown in the following Table 2. Here, scores are as shown in Table 1.

[Table 2]

| | Base sequence (5'→3') |
|---|---|
| SEQ ID NO: 1: | CGCTCTTCCGATCTCTGGTTCGATGCGGACCTTCTGG |
| SEQ ID NO: 2: | CGCTCTTCCGATCTGACTCTCCCACATCCGGCTATGG |

From the base sequences of SEQ ID No: 1 and SEQ ID No: 2, a dot matrix shown in Table 3 is generated. Specifically, the base sequence of SEQ ID No: 1 is arranged from the left to the right in an orientation of 5' to 3' and the base sequence of SEQ ID No: 2 is arranged from the bottom to the top in an orientation of 5' to 3'. "•" is filled in a grid of which bases are complementary to each other, and a dot matrix shown in Table 3 is obtained.

[Table 3]

From the dot matrix shown in Table 3, Alignment (pairwise alignment) of partial sequences shown in the following Table 4 is obtained (refer to a diagonal line portion of Table 3). In Table 4, matches are represented by "1."

[Table 4]

| Partial sequence from SEQ ID NO: 1: | 5' - A C C T T - C T G G - 3' |
| |       I    I      I    I  I |
| Partial sequence from SEQ ID NO: 2: | 3' - G G T A T C G G C C - 5' |

In this (pairwise) alignment, there are five matches, there are four mismatches, and there is one indel (gap).

Therefore, a local alignment score based on this (pairwise) alignment is (+ 1) × 5 + (-1) × 4 + (-1) × 1 = ±0.

The alignment (pairwise alignment) can be obtained not only through the dot matrix method exemplified herein, but also through a dynamic programming method, a word method, or various other methods.

<<First Stage Selection Step>>

In the present specification, the first stage selection step S104 (Fig. 2), the first step of first stage selection S204 and the second step of first stage selection S214, and the first first-stage selection step S304 and the second first-stage selection step S314 will be collectively referred to as "first stage selection step" in some cases.

(First Aspect: First Stage Selection Step S104)

This step is shown as "first stage selection" (S104) in Fig. 2.

In the first aspect, the (d) first stage selection step is a step of performing first stage selection of the base sequence of the primer candidate for PCR amplifying the target region based on the local alignment score.

(Second Aspect: First Step of First Stage Selection S204 and Second Step of First Stage Selection S214)

These steps are shown as "first step of first stage selection" (S204) and "second step of first stage selection" (S214) in Fig. 3.

In the second aspect, the ($d_1$) first step of first stage selection is a step of performing first stage selection of the base sequence of the primer candidate for PCR amplifying the first target region based on the local alignment score. The ($d_2$) second step of first stage selection is a step of performing first stage selection of the base sequence of the primer candidate for PCR amplifying the second target region based on the local alignment score.

(Third Aspect: First First-Stage Selection Step S304 and Second First-Stage Selection Step S314)

These steps are shown as "first first-stage selection" (S304) and "second first-stage selection" (S314) in Fig. 4.

In the third aspect, the (d-1) first first-stage selection step is a step of performing first stage selection of the base sequence of the primer candidate for PCR amplifying the first target region based on the local alignment score. The (d-2) second first-stage selection step is a step of performing first stage selection of the base sequence of the primer candidate for PCR amplifying the second target region based on the local alignment score.

(First Stage Selection Method)

A threshold value (first threshold value) of the local alignment score is predetermined.

In a case where a local alignment score of a pair of two base sequences is less than the first threshold value, it is determined that the pair of these two base sequences has low dimer formability, and the following step is performed. In contrast, in a case where a local alignment score of a pair of two base sequences is greater than or equal to the first threshold value, it is determined that the pair of these two base sequences has high dimer formability, and the following step is not performed on the pair. The first threshold value is not particularly limited and can be appropriately set. For example, the first threshold value may be set according to PCR conditions such as an amount of genomic DNA used as a template for a polymerase chain reaction.

Here, in the example shown in the above-described "local alignment step," a case where the first threshold value is set to "+ 3" is considered.

In the above-described example, the local alignment score is "$\pm 0$" and is less than "+ 3" which is the first threshold value. Therefore, it is possible to determine that the pair of the base sequences of SEQ ID No: 1 and SEQ ID No: 2 has low dimer formability.

The present step is performed on each of combinations which are obtainable with local alignment scores calculated in the local alignment step S103, the first step of local alignment S203, the second step of local alignment S213, the first local alignment step S303, or the second local alignment step S313.

«Global Alignment Step»

In the present specification, the global alignment step S105 (Fig. 2), the first step of global alignment S205 and the second step of global alignment S215 (Fig. 3), and the first global alignment step S305 and the second global alignment step S315 (Fig. 4) will be collectively referred to as a "global alignment step" in some cases.

(First Aspect: Global Alignment Step S105)

This step is shown as "global alignment" (S105) in Fig. 2.

In the first aspect, the (e) global alignment step is a step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step.

(Second Aspect: First Step of Global Alignment S205 and Second Step of Global Alignment S215)

These steps are shown as "first step of global alignment" (S205) and "second step of global alignment" (S215) in Fig. 3.

In the second aspect, the ($e_1$) first step of global alignment is a step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection. The ($e_2$) second step of global alignment is a step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed.

(Third Aspect: First Global Alignment Step S305 and Second Global Alignment Step S315)

These steps are shown as "first global alignment" (S305) and "second global alignment" (S315) in Fig. 4.

In the third aspect, the (e-1) first global alignment step is a step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step. The (e-2) second global alignment step is a step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed.

(Global Alignment Method)

The global alignment score is obtained by selecting two primers from the group consisting of all of the primer candidates generated in the "primer candidate base sequence generation step" (in a case where the "local alignment step" and the "first stage selection step" are performed first, and in a case where there are combinations of primer candidates in which a local alignment score is less than the first threshold value, all of the primer candidates included in the combinations thereof), and all of the primers that have already been employed (limited to cases where primers that have already been employed are present); and performing global alignment in a pairwise manner for the base sequence with a predetermined sequence length which includes 3' terminals.

A combination of base sequences to be subjected to global alignment may be a combination selected while allowing overlapping, or may be a combination selected without allowing overlapping. However, in a case where formability of a primer-dimer between primers of an identical base sequence has not yet been evaluated, the combination selected while allowing overlapping is preferable.

The total number of combinations is "$_xH_2 = {}_{x+1}C_2 = (x+1)! / 2(x-1)!$" in a case where the selection is performed while allowing overlapping, and is "$_xC_2 = x(x-1) / 2$" in a case where the selection is performed without allowing overlapping, in which the total number of base sequences for performing global alignment is set to be x.

Global alignment is an alignment which is performed on the entire sequence and in which it is possible to check complementarity of the entire sequence.

However, here, the "entire sequence" refers to the entirety of a base sequence which has a predetermined sequence length and includes the 3' terminal of a base sequence of a primer candidate.

Global alignment may be performed by inserting a gap. The gap means insertion and/or deletion (indel) of a base.

In addition, in the global alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, scores may be set as in Table 1. "-" in Table 1 represents a gap (insertion and/or deletion (indel)).

For example, it is considered that global alignment is performed on three bases (refer to portions with capital letters and correspond to the "base sequence which has a predetermined sequence length and includes the 3' terminal") at the 3' terminal of each base sequence of SEQ ID No: 1 and SEQ ID No: 2 shown in the following Table 5. Here, the scoring system is as shown in Table 1.

[Table 5]

| | Base sequence (5'→3') |
|---|---|
| SEQ ID NO: 1: | cgctcttccgatctctggttcgatgcggaccttcTGG |
| SEQ ID NO: 2: | cgctcttccgatctgactctcccacatccggctaTGG |

In a case of performing global alignment on base sequences of the three bases (portion with capital letters) at the 3' terminal of the base sequence of SEQ ID No: 1 and the three bases (portion with capital letters) at the 3' terminal of SEQ ID No: 2 such that the score becomes a maximum, it is possible to obtain alignment (pairwise alignment) shown in the following Table 6.

[Table 6]

| 3' Terminal 3 bases of SEQ ID NO: 1: | 5'- | T | G | G | -3' |
|---|---|---|---|---|---|
| 3' Terminal 3 bases of SEQ ID NO: 2: | 3'- | G | G | T | -5' |

In this (pairwise) alignment, there are three mismatches, and there is no matches and indels (gap).
Therefore, a global alignment score based on this (pairwise) alignment is (+ 1) $\times$ 0 + (-1) $\times$ 3 + (-1) $\times$ 0 = -3.
The alignment (pairwise alignment) can be obtained through the dot matrix method a dynamic programming method, a word method, or various other methods.

«Second Stage Selection Step»

In the present specification, the second stage selection step S106 (Fig. 2), the first step of second stage selection S206 and the second step of second stage selection S216 (Fig. 3), and the first second-stage selection step S306 and the second second-stage selection step S316 (Fig. 4) will be collectively referred to as a "second stage selection step" in some cases.

(First Aspect: Second Stage Selection Step S106)

This step is shown as "second stage selection" (S106) in Fig. 2.
In the first aspect, the (f) second stage selection step is a step of performing second stage selection of the base sequence of the primer candidate for PCR amplifying the target region based on the global alignment score.

(Second Aspect: First Step of Second Stage Selection S206 and Second Step of Second Stage Selection S216)

These steps are shown as "first step of second stage selection" (S206) and "second step of second stage selection" (S216) in Fig. 3.
In the second aspect, the ($f_1$) first step of second stage selection is a step of performing second stage selection of the base sequence of the primer candidate for PCR amplifying the first target region based on the global alignment score. The ($f_2$) second step of second stage selection is a step of performing second stage selection of the base sequence of the primer candidate for PCR amplifying the second target region based on the global alignment score.

(Third Aspect: First Second-Stage Selection Step S306 and Second Second-Stage Selection Step S316)

These steps are shown as "first second-stage selection" (S306) and "second second-stage selection" (S316) in Fig. 4.
In the third aspect, the (f-1) first second-stage selection step is a step of performing second stage selection of the base sequence of the primer candidate for PCR amplifying the first target region based on the global alignment score. The (f-2) second second-stage selection step is a step of performing second stage selection of the base sequence of the primer candidate for PCR amplifying the second target region based on the global alignment score.

(Second Stage Selection Method)

A threshold value (also referred to as a "second threshold value") of the global alignment score is predetermined.

In a case where a global alignment score is less than the second threshold value, it is determined that a combination of these two base sequences has low dimer formability, and the following step is performed.

In contrast, in a case where a global alignment score is greater than or equal to the second threshold value, it is determined that a combination of these two base sequences has high dimer formability, and the following step is not performed on the combination.

The second threshold value is not particularly limited and can be appropriately set. For example, the second threshold value may be set according to PCR conditions such as an amount of genomic DNA used as a template for a polymerase chain reaction.

It is possible to set the global alignment score obtained by performing pairwise global alignment on a base sequence which has a predetermined number of bases and includes the 3' terminal of a base sequence of each primer to be less than the second threshold value by setting a base sequence with several bases from the 3' terminal of a primer as an identical base sequence.

Here, in the example shown in the above-described "global alignment step," a case where the second threshold value is set to "+ 3" is considered.

In the above-described example, the global alignment score is "-3" and is less than "+3" which is the second threshold value. Therefore, it is possible to determine that the pair of the base sequences of SEQ ID No: 1 and SEQ ID No: 2 has low dimer formability.

The present step is performed on each of combinations which are obtainable with global alignment scores calculated in the global alignment step S105, the first step of global alignment S205, the second step of global alignment S215, the first global alignment step S305, or the second global alignment step S315.

In addition, in order to reduce the amount of calculation, it is preferable to first perform both steps of the "global alignment step" and the "second stage selection step" and to perform both steps of the "local alignment step" and the "first stage selection step" in a combination of the base sequences of the primers, which has passed the "second stage selection step." Particularly, as the number of target regions and the number of base sequences of primer candidates are increased, the effect of reducing the amount of calculation is increased, and it is possible to speed up the overall processing.

This is because the amount of calculation of a global alignment score is smaller than that of a local alignment score which is obtained by searching a partial sequence with high complementarity from the entire base sequence under the condition that the base sequence includes the 3' terminal and it is possible to speed up the processing since global alignment is performed on a base sequence with a short length called a "predetermined sequence length" in the "global alignment step." It is known that the global alignment is faster than the local alignment in a case of alignment with respect to a sequence having an identical length in a well-known algorithm.

«Amplification Sequence Length-Checking Step»

An amplification sequence length-checking step of calculating the distance between ends of base sequences of primer candidates for which it has been determined that formability of a primer-dimer is low in the "first stage selection step" and "second stage selection step" on chromosomal DNA regarding combinations of the base sequences of the primer candidates, and determining whether the distance is within a predetermined range may be performed.

In a case where the distance between the ends of the base sequences is within the predetermined range, it is possible to determine that there is a high possibility that the combinations of the base sequences of the primer candidates can appropriately amplify a target region. The distance between the ends of the base sequences of the primer candidates is not particularly limited, and can be appropriately set in accordance with the PCR condition such as the type of enzyme (DNA polymerase). For example, the distance between the ends of the base sequences of the primer candidates can be set to be within various ranges such as a range of 100 to 200 bases (pair), a range of 120 to 180 bases (pair), a range of 140 to 180 bases (pair) a range of 140 to 160 bases (pair), and a range of 160 to 180 bases (pair).

«Primer Employment Step»

In the present specification, the primer employment step S107 (Fig. 2), the first step of primer employment S207 and the second step of primer employment S217 (Fig. 3), and the first primer employment step S307 and the second primer employment step S317 (Fig. 4) will be collectively referred to as a "primer employment step" in some cases.

(First Aspect: Primer Employment Step S107)

This step is shown as "primer employment" (S107) in Fig. 2.

In the first aspect, the (g) primer employment step is a step of employing the base sequence of the primer candidate which is selected in both of the first stage selection step and the second stage selection step as the base sequence of the primer for PCR amplifying the target region.

(Second Aspect: First Step of Primer Employment S207 and Second Step of Primer Employment S217)

These steps are shown as "first step of primer employment" (S207) and "second step of primer employment" (S217) in Fig. 3.

In the second aspect, the (g$_1$) first step of primer employment is a step of employing the base sequence of the primer candidate which is selected in both of the first step of first stage selection and the first step of second stage selection as a base sequence of a primer for PCR amplifying the first target region. The (g$_2$) second step of primer employment is a step of employing the base sequence of the primer candidate which is selected in both of the second step of first stage selection and the second step of second stage selection as a base sequence of a primer for PCR amplifying the second target region.

(Third Aspect: First Primer Employment Step S307 and Second Primer Employment Step S317)

These steps are shown as "first primer employment" (S307) and "second primer employment" (S317) in Fig. 4.

In the third aspect, the (g-1) first primer employment step is a step of employing the base sequence of the primer candidate which is selected in both of the first first-stage selection step and the first second-stage selection step as the base sequence of the primer for PCR amplifying the first target region. The (g-2) second primer employment step is a step of employing the base sequence of the primer candidate which is selected in both of the second first-stage selection step and the second second-stage selection step as the base sequence of the primer for PCR amplifying the second target region.

(Primer Employment Method)

In the primer employment step, the base sequence of the primer candidate, in which a local alignment score obtained by performing pairwise local alignment on a base sequence of each primer candidate under a condition that a partial sequence to be subjected to comparison includes the 3' terminal of the base sequence is less than the first threshold value, and a global alignment score obtained by performing pairwise global alignment on a base sequence which has a predetermined number of bases and includes the 3' terminal of the base sequence of each primer candidate is less than the second threshold value, is employed as the base sequence of the primer for amplifying a target region.

For example, it is considered that base sequences of SEQ ID No: 1 and SEQ ID No: 2 shown in Table 7 are employed as base sequences of primers for amplifying a target region.

[Table 7]

| Base sequence (5'→3') | |
|---|---|
| SEQ ID NO: 1: | CGCTCTTCCGATCTCTGGTTCGATGCGGACCTTCTGG |
| SEQ ID NO: 2: | CGCTCTTCCGATCTGACTCTCCCACATCCGGCTATGG |

As already described, the local alignment score is "±0" and thus is less than "+3" which is the first threshold value, and the global alignment score is "-3" and thus is less than "+3" which is the second threshold value.

Accordingly, it is possible to employ the base sequence of the primer candidate represented by SEQ ID No: 1 and the base sequence of primer candidate represented by SEQ ID No: 2 as base sequences of primers for amplifying a target region.

«Primer Design of Other Objective Regions»

After employing a primer for one objective region, primers for another objective region may further be designed.

In the first aspect, in the primer candidate base sequence generation step S102, in a case where a base sequence of a primer candidate for an objective region for which a primer is to be designed is already generated, steps are carried out from the local alignment step S103. In a case where a base sequence of a primer candidate for the next objective region is not yet generated, because the next objective region is not selected in the target region selection step S101, the next objective region is selected in the target region selection step S101, and a base sequence of a primer candidate for the objective region is generated in the primer candidate base sequence generation step S102, and then steps subsequent to the local alignment step S103 are carried out.

In the second aspect, steps are repeated from the second step of target region selection S211.

In the third aspect, since the base sequence of the primer candidate for the objective region already selected in the target region multiple selection step S301 is already generated in the primer candidate base sequence multiple generation step S302, steps are repeated from the second local alignment step S313.

<<Characteristic Points in Design of Primers and the Like>>

Characteristic points in the design of the primers and the like after selecting the objective region in general are as follows: obtaining primer groups which include target regions as targets to be amplified and do not become complementary to each other, by selecting a plurality of specific target regions, searching vicinity base sequences, checking complementarity with each extracted primer set, and selecting base sequences having low complementarity.

Characteristic points in checking of the complementarity of a base sequence of a primer are that primer groups are generated such that complementarity of a whole sequence becomes low using local alignment and the complementarity with respect to ends of a base sequence of a primer becomes low using global alignment.

[Method for Identifying Individual]

A method for identifying an individual of the embodiment of the present invention uses the above-described method for discriminating an origin of human genomic DNA of 100 pg or less.

Among human genes, a length of Short Tandem Repeat (STR) which is a locus with the number of repetition of short repetition units, and Single Nucleotide Polymorphisms (SNP) which is a locus having information that one base has been replaced with another base are known as different regions depending on individuals, and individuals can be identified by examining a length of a plurality of STR loci and sequences of SNP loci.

For example, it is possible to acquire base sequence information with high accuracy even from a trace amount of DNA contained in hair, blood stains, or the like of a subject to be subjected to DNA examination in identifying a body at the time of a disaster or conducting a criminal investigation. There are very few cases where articles left on the scene or the like of an individual who is a subject to be subjected to individual identification contain somatic cells to be obtained generally, and there is even a case where about 10 cells are recovered as the number of cells. In such a case, in the DNA amplification technique of the related art, a method of amplifying DNA to be analyzed by the whole genome amplification method on an amount of DNA extracted from about 10 or less cells and mixed, and indirectly determining a base sequence of this amplification product has been generally used; however, in this method, conditions that a region which is not amplified due to unevenness of the amplified region is generated, and an amplification product that does not reflect correct base sequence information is obtained due to amplification error of DNA synthetase, and therefore it is not always possible to acquire highly accurate sequence information have been pointed out as problems. In addition, even in a case where PCR is performed directly from a trace amount of DNA without performing whole genome amplification, conditions in which unevenness due to excessive amplification reaction caused by an extremely small amount of template DNA, and nonspecific amplification (primer-dimer) between primers used for obtaining base sequence information occur, and therefore accurate base sequence information cannot be acquired exist as problems.

DNA to be analyzed at the time of a disaster or criminal investigation is not always genomic DNA with a complete form, and is DNA exposed from the cell nucleus and thus is fragmented by physical actions or chemical actions represented by ultraviolet rays in many cases. Gene sequence information can also be obtained from a trace amount of such fragmented DNA.

According to the method for identifying an individual of the embodiment of the present invention, it is possible to acquire base sequence information with high accuracy even from a trace amount of DNA contained in hair, blood stains, or the like of a subject to be subjected to DNA examination in identifying a body at the time of a disaster or conducting a criminal investigation. There are very few cases where articles left on the scene or the like of an individual who is a subject to be subjected to individual identification contain somatic cells to be obtained generally, and there is even a case where about 10 cells are recovered as the number of cells. Even in such a case, according to the present invention, it is possible to perform individual identification with higher accuracy.

[Method for Analyzing Level of Engraftment of Hematopoietic Stem Cell]

A method for analyzing a level of engraftment of hematopoietic stem cells of the embodiment of the present invention uses the above-described method for discriminating an origin of human genomic DNA of 100 pg or less.

An examination called a chimerism test is an examination used as an index for checking how much hematopoietic stem cells transplanted to the body of a patient with a disorder making normal blood cells difficult to be generated have engrafted, and even in such an examination, it is possible to acquire base sequence information with high accuracy from a small amount of DNA.

Bone marrow transplantation is performed as a radical treatment method for diseases such as tumors of hematopoietic organs and aplastic anemia. In this case, checking the engraftment of bone marrow cells of a healthy donor (donor) and extermination of cells of a patient (recipient) after transplantation is extremely important for judging the success or failure of transplantation and judging prognosis. After transplantation, there is a period in which cells derived from a patient and cells derived from a healthy donor are mixed in the body of the patient, which is referred to as a mixed chimeric state. An examination that numerically quantifies this state is the chimerism test.

In the chimerism test, cells are divided into T lymphocytes and cells other than T lymphocytes so as to be analyzed, respectively.

Currently, a mainly used chimerism examination method is a STR method, and STR of the cells is examined. In advance, STR of a donor providing hematopoietic stem cells and STR of a patient receiving transplantation of hematopoietic stem cells are examined, and STR loci having different lengths from each other are confirmed. After transplantation of hepatocytes, by obtaining cells having a plurality of nuclei in the blood and mixing the extracted DNA so as to perform observation, it is possible to confirm that the hematopoietic stem cells have firmly engrafted when information having the length of STR derived from transplanted cells of the donor finally becomes 100%.

In a case of examining SNP, the number of SNP sequences at the corresponding SNP locus is decoded by a next generation sequencer, and when information of SNP of the donor reaches 100%, it is possible to check a state where the hematopoietic stem cells have firmly engrafted.

In a case of identification by using information of SNP, the accuracy of identification increases as the number of SNP loci to be compared increases. The probability (P) of having the same SNP sequence among individuals is calculated, and found to be $P = 5.2 \times 10^{-1}$ for one position of SNP sequence, $P = 3.8 \times 10^{-2}$ for 5 positions of SNP sequences, $P = 1.45 \times 10^{-3}$ for 10 positions of SNP sequences, $P = 5.5 \times 10^{-5}$ for 15 positions of SNP sequences, and $P = 2.09 \times 10^{-6}$ for 20 positions of SNP sequences. That is, in a case where about 20 positions of SNP sequences can be compared, a level of engraftment of hematopoietic stem cells can be identified with high accuracy.

A method for detecting T lymphocytes includes a step in which information derived from cells of a sample liquid is obtained by a flow cytometry method, and based on the obtained information, objective cells are fractionated into containers in which wells having openings are arranged. Cells fractionated into containers are captured, and based on the images, T lymphocyte candidate cells are determined. Dyeing is performed before fractionating with a flow cytometer. First, a sample to be analyzed containing objective cells is prepared. The sample to be analyzed is mixed with, for example, a hemolytic agent and a fluorescently labeled antibody used for immunostaining, and incubated, and therefore cells are immunostained. A sample liquid S is prepared by immunostaining the cells. Blood cells are irradiated with laser light or the like from a light source. Fluorescent labeling by immunostaining of the blood cells is excited by irradiation with laser light, and the blood cells emit fluorescence by fluorescent labeling by immunostaining. This fluorescence intensity is detected by a detector. On the basis of detected information, cells in which ultrasound is applied to the flow cell are charged positively or negatively. In a case where cells passes through deflecting electrode plates, one cell is basically fractionated in one well of the container by attracting charged liquid droplets to one of the deflecting electrode plates.

Hereinafter, the present invention will be described in more detail using Examples, but is not limited to these Examples.

Examples

[Example 1]

1. Preparation of Analytical DNA

(1) Genomic DNA

Genomic DNA extracted from human cultured cells and mixed was diluted with DNase (DNA-degrading enzyme)-free ultrapure water, and therefore genomic DNA aqueous solutions in which genomic DNA concentrations were 2.5 ng/$\mu$L, 250 pg/$\mu$L, 125 pg/$\mu$L, 25 pg/$\mu$L, 12.5 pg/$\mu$L, and 2.5 pg/$\mu$L were prepared.

4 $\mu$L of the genomic DNA aqueous solution of each concentration was added to each 0.2 mL PCR tube. Amounts of genomic DNA contained in each PCR tube were 10 ng, 1 ng, 500 pg, 100 pg, 50 pg, and 10 pg, respectively.

(2) Hair DNA

A single hair was collected from a male volunteer (worker A).

The collected hair was cut into a hair root part and hair shaft, and this hair root part was added to a 0.2 mL PCR tube.

An amount of genomic DNA contained in the PCR tube to which the hair root part was added was about 50 pg.

2. Protein Lysis Treatment

To a 0.2 mL PCR tube containing 4 μL of analytical DNA, 5 μL of a cell lysis buffer (10 mmol/L Tris-HCl pH 7.5, 0.5 mmol/L ethylenediamine tetraacetic acid, 20 mmol/L potassium chloride, 0.007% (w/v) sodium dodecyl sulfate, 13.3 μg/mLproteinase K) was added.

Each PCR tube was heated at 50°C for 60 minutes, and protein was dissolved with Proteinase K. Next, each tube was heated at 95°C for 5 minutes to inactivate Proteinase K.

Analytical DNA subjected to the protein lysis treatment (hereinafter sometimes referred to as "protein lysate") was stored at 12°C until next use.

3. PCR Amplification of Analytical DNA

(1) Preparation of Primer

The primers used for multiplex PCR were prepared based on base sequence information of genes or chromosomal sites of 647 positions containing human single nucleotide polymorphisms (SNPs).

Local alignment is shown in Table 8 and global alignment is shown in Table 9 for primer 2001-F (SEQ ID NO: 1) and primer 2001-R (SEQ ID NO: 2).

Scoring was set as match = +1, mismatch = -1, and gap (indel) = -1, and a sum of the scores of each position was taken as the alignment score.

A threshold value of the local alignment score was set to "+3", and the local alignment score of the primers used for multiplex PCR was required to be less than this threshold value.

In addition, a threshold value of the global alignment score was set to "±0", and the global alignment score of the primers used for multiplex PCR was required to be less than this threshold value.

[Table 8]

| Partial sequence from primer 2001-F: | 5'- | A | C | C | T | T | - | C | T | G | G | -3' |
|---|---|---|---|---|---|---|---|---|---|---|---|---|
| | | | | | | | | | | | | |
| Partial sequence from primer 2001-R: | 3'- | G | G | T | A | T | C | G | G | C | C | -5' |

The local alignment score is match (+1) × 5 + mismatch (-1) × 4 + indel (-1) × 1 = ±0.

Therefore, the local alignment score is less than the threshold value.

[Table 9]

| 3' Terminal 3 bases of primer 2001-F: | 5'- | T | G | G | -3' |
|---|---|---|---|---|---|
| 3'Terminal 3 bases of primer 2001-R: | 3'- | G | G | T | -5' |

The global alignment score is match (+1) × 0 + mismatch (-1) × 3 + indel (-1) × 0 = -3.

Therefore, the global alignment score is less than the threshold value.

In a part of the prepared primers, names of the primers of each primer pair, the base sequence, and the sequence length are shown in Table 10, and an amplification site and amplification product size of each primer pair are shown in Table 11.

[Table 10]

| ID | Primer pair | Primer | | | SEQ ID NO |
|---|---|---|---|---|---|
| | | Name | Base sequence (5'→3') | Sequence length | |
| 1 | 2001 | 2001-F | CGCTCTTCCGATCTCTGgttcgatgcggaccttctgg | 37mer | 1 |
| | | 2001-R | CGCTCTTCCGATCTGACtctcccacatccggctatgg | 37mer | 2 |
| 2 | 2002 | 2002-F | CGCTCTTCCGATCTCTGtttccccgaccataagcttg | 37mer | 3 |
| | | 2002-R | CGCTCTTCCGATCTGACatacagggctgagagattgg | 37mer | 4 |
| 3 | 2003 | 2003-F | CGCTCTTCCGATCTCTGtgataaggtccgaactttgg | 37mer | 5 |
| | | 2003-R | CGCTCTTCCGATCTGACgcgactgcaagagattcgtg | 37mer | 6 |
| 4 | 2004 | 2004-F | CGCTCTTCCGATCTCTGatttgctgctgaccagggtg | 37mer | 7 |
| | | 2004-R | CGCTCTTCCGATCTGACaggtacagcttcccatctgg | 37mer | 8 |
| 5 | 2005 | 2005-F | CGCTCTTCCGATCTCTGccgtgtgtgagattctcgtg | 37mer | 9 |
| | | 2005-R | CGCTCTTCCGATCTGACactgctcagggtcctctgtg | 37mer | 10 |
| 74 | 2078 | 2078-F | GGCTCTTCCGATCTCTGaggttctgctcgttggcttg | 37mer | 11 |
| | | 2078-R | CGCTCTTCCGATCTGACccaagaaggacacggattgg | 37mer | 12 |
| 75 | 2079 | 2079-F | CGCTCTTCCGATCTCTGagcttcccgggaaattagtg | 37mer | 13 |
| | | 2079-R | CGCTCTTCCGATCTGACgaattttaatcgcccctgtg | 37mer | 14 |
| 76 | 2060 | 2080-F | CGCTCTTCCGATCTCTGggactgcttagatgccgtgg | 37mer | 15 |
| | | 2080-R | CGCTCTTCCGATCTGACagtcaaaagcatgtcagtgg | 37mer | 16 |
| 77 | 2081 | 2081-F | CGCTCTTCCGATCTCTGctctgtggagtccgtgatgg | 37mer | 17 |
| | | 2081-R | CGCTCTTCCGATCTGACatgcagtcacagtggtatgg | 37mer | 18 |
| 78 | 2082 | 2082-F | CGCTCTTCCGATCTCTGggtaatgctgcaagctctgg | 37mer | 19 |
| | | 2082-R | CGCTCTTCCGATCTGACcctaggggatcaagatgtgg | 37mer | 20 |
| 123 | 2128 | 2128-F | CGCTCTTCCGATCTCTGgcaagagtctggctttttg | 37mer | 21 |
| | | 2128-R | CGCTCTTCCGATCTGACgggatcaggtactgccgttg | 37mer | 22 |
| 124 | 2129 | 2129-F | CGCTCTTCCGATCTCTGcaaggtgctacatgtgctgg | 37mer | 23 |
| | | 2129-R | CGCTCTTCCGATCTGACgctttgcaggggaatgtttg | 37mer | 24 |
| 125 | 2130 | 2130-F | CGCTCTTCCGATCTCTGgagcagcgtaccattgggtg | 37mer | 25 |
| | | 2130-R | CGCTCTTCCGATCTGACcagtgttctcgctcatgtgg | 37mer | 26 |
| 126 | 2131 | 2131-F | CGCTCTTCCGATCTCTGtgtctcccccttttagttg | 37mer | 27 |
| | | 2131-R | CGCTCTTCCGATCTGACtttacctggctttggagttg | 37mer | 28 |
| 127 | 2132 | 2132-F | CGCTCTTCCGATCTCTGgtcagcaagttggctactgg | 37mer | 29 |
| | | 2132-R | CGCTCTTCCGATCTGACagccttaggctcccatggtg | 37mer | 30 |
| 164 | 2169 | 2169-F | CGCTCTTCCGATCTCTGagactcactccacgtgtgtg | 37mer | 31 |
| | | 2169-R | CGCTCTTCCGATCTGACagaacccagtggtgaatttg | 37mer | 32 |
| 165 | 2170 | 2170-F | CGCTCTTCCGATCTCTGcttccccttctgtgggtgtg | 37mer | 33 |
| | | 2170-R | CGCTCTTCCGATCTGACaggataaaacaatgggttgg | 37mer | 34 |
| 166 | 2171 | 2171-F | CGCTCTTCCGATCTCTGctgttgccgtctcttcatgg | 37mer | 35 |
| | | 2171-R | CGCTCTTCCGATCTGACacctctggaggaagttgttg | 37mer | 36 |

(continued)

| ID | Primer pair | Primer | | | SEQ ID NO |
|---|---|---|---|---|---|
| | | Name | Base sequence (5'→3') | Sequence length | |
| 167 | 2173 | 2173-F | CGCTCTTCCGATCTCTGgaactccttgtggcggcttg | 37mer | 37 |
| | | 2173-R | CGCTCTTCCGATCTGACcctgcaagaaggtcttatgg | 37mer | 38 |
| 168 | 2175 | 2175-F | CGCTCTTCCGATCTCTGctccatggcttggatcttgg | 37mer | 39 |
| | | 2175-R | CGCTCTTCCGATCTGACagcgcctggacagctatgtg | 37mer | 40 |
| 169 | 2176 | 2176-F | CGCTCTTCCGATCTCTGtacgccaggtgtctcgcttg | 37mer | 41 |
| | | 2176-R | CGCTCTTCCGATCTGACtccagataaaggcggctttg | 37mer | 42 |

[Table 11]

| ID | Primer pair | Amplification site | | | | Amplification product size |
|---|---|---|---|---|---|---|
| | | Chromosome | Start site | Termination site | SNP position | |
| 1 | 2001 | chr13 | 20763333 | 20763509 | 2076380 | 177bp |
| 2 | 2002 | chr13 | 21205086 | 21205235 | 21205192 | 150bp |
| 3 | 2003 | chr13 | 21619945 | 21620115 | 21620085 | 171bp |
| 4 | 2004 | chr13 | 23898446 | 23898625 | 23898509 | 180bp |
| 5 | 2005 | chr13 | 24797765 | 24797943 | 24797913 | 179bp |
| 74 | 2078 | chr18 | 346690 | 346870 | 346821 | 181bp |
| 75 | 2079 | chr18 | 3075668 | 3075830 | 3075712 | 163bp |
| 76 | 2080 | chr18 | 3188929 | 3189087 | 3188976 | 159bp |
| 77 | 2081 | chr18 | 3457482 | 3457660 | 3457539 | 179bp |
| 78 | 2082 | chr18 | 5416011 | 5416189 | 5416160 | 17 9bp |
| 123 | 2128 | chr21 | 16340252 | 16340421 | 16340289 | 170bp |
| 124 | 2129 | chr21 | 28212733 | 28212890 | 28212760 | 158bp |
| 125 | 2130 | chr21 | 28305169 | 28305321 | 28305212 | 153bp |
| 126 | 2131 | chr21 | 30408533 | 30408700 | 30408670 | 168bp |
| 127 | 2132 | chr21 | 30464815 | 30464979 | 30464966 | 165bp |
| 164 | 2169 | chrX | 2779532 | 2779679 | 2779570 | 148bp |
| 165 | 2170 | chrX | 2951372 | 2951551 | 2951434 | 180bp |
| 166 | 2171 | chrX | 3240902 | 3241080 | 3241050 | 179bp |
| 167 | 2173 | chrX | 6995381 | 6995555 | 6995417 | 175bp |
| 168 | 2175 | chrX | 14027113 | 14027264 | 14027177 | 152bp |
| 169 | 2176 | chrX | 16168334 | 16168510 | 16168467 | 177bp |

(2) Confirmation of Amplification through Singleplex PCR

In order to confirm whether or not a desired amplification site can be specifically amplified using the prepared primers, singleplex PCR was performed for each primer pair.

a) Preparation of reaction liquid

The following reaction liquid was prepared.

| | |
|---|---|
| Genomic DNA (0.5 ng/μL) | 2 μL |
| Primers | 2 μL for each |
| Multiplex PCR Mix 1 | 0.125 μL |
| Multiplex PCR Mix 2 | 12.5 μL |
| Sterilized distilled water | up to 25 μL |

The genomic DNA is genomic DNA extracted from human cultured cells, and Multiplex PCR Mix 1 and Multiplex PCR Mix 2 are reagents contained in TaKaRa Multiplex PCR Assay Kit (manufactured by Takara Bio Inc.).

b) Reaction conditions for singleplex PCR

As reaction conditions for singleplex PCR, 30 cycles of thermal cycle: [thermal denaturation (94°C, 30 seconds) - annealing (60°C, 90 seconds) - extension (72°C, 30 seconds)] were carried out after initial thermal denaturation (94°C, 60 seconds).

c) Confirmation of PCR Amplification Product

A portion of the reaction liquid after completion of the reaction was subjected to agarose gel electrophoresis, and the presence or absence and size of the PCR amplification product were confirmed.

Specific amplification by singleplex PCR could be confirmed for 647 pairs of primers.

(3) Amplification by Multiplex PCR

a) Preparation of reaction liquid

The following reaction liquid was prepared.

| | |
|---|---|
| Template DNA | 9 μL |
| Primer · mix | 4 μL |
| Multiplex PCR Mix 1 | 0.125 μL |
| Multiplex PCR Mix 2 | 12.5 μL |
| Sterilized distilled water | up to 26 μL |

The template DNA is the "protein lysate" stored in "2. Protein Lysis Treatment" described above, the primer · mix is prepared by mixing 647 pairs of primer pairs so that a final concentration of each primer became 50 nmol/L, and Multiplex PCR Mix 1 and Multiplex PCR Mix 2 are reagents contained in TaKaRa Multiplex PCR Assay Kit (Takara Bio Inc.).

b) Reaction conditions for Multiplex PCR

As reaction conditions for Multiplex PCR, 32 cycles of thermal cycle: [thermal denaturation (94°C, 30 seconds) - annealing (56.7°C, 600 seconds) - extension (72°C, 30 seconds)] were carried out after initial thermal denaturation (94°C, 60 seconds).

c) Purification of PCR Reaction Product

The PCR reaction product obtained by multiplex PCR was purified according to the attached manual (Instructions For Use) using Agencourt AMPure XP (Beckman Coulter).

Specifically, 45 μL of the AMPure XP reagent was added to 25 μL of the PCR reaction liquid, mixed thoroughly, and allowed to stand at room temperature for 5 minutes to bind the PCR reaction product to magnetic beads.

Next, the magnetic beads to which the PCR reaction product was bound were separated by magnetic force of a magnet stand (MagnaStand, manufactured by Japan Genetics Co., Ltd.) to remove contaminants.

After washing the magnetic beads to which the PCR reaction product was bound twice with 70% (v/v) ethanol, DNA bound to the magnetic beads was eluted with 40 μL of Tris-HCl buffer solution.

4. DNA Sequencing

(1) Preparation of Sequence Library Mix

In order to perform dual index sequencing using a next generation sequencer (Miseq, Illumina Co.), two kinds of adapters including a flow cell binding sequence (P5 sequence or P7 sequence), an index sequence for sample identification, and a sequence primer binding sequence were added to both ends of a DNA fragment obtained by multiplex PCR, respectively.

Specifically, addition of sequences was performed to both ends by performing PCR using the Multiplex PCR Assay kit and using 1.25 μM of each of primers D501 to D508 and D701 to D712 shown in Table 12.

As PCR conditions, 5 cycles of thermal cycle: [thermal denaturation (94°C, 45 seconds) - annealing (50°C, 60 seconds) - extension (72°C, 30 seconds)] were carried out after initial thermal denaturation (94°C, 3 minutes), and then 11 cycles of thermal cycle: [thermal denaturation (94°C, 45 seconds) - annealing (55°C, 60 seconds) - extension (72°C, 30 seconds)] were carried out.

[Table 12]

| Primer | | | SEQ ID NO |
|---|---|---|---|
| Name | Base sequence (5'→3') | Sequence length | |
| D501 | AATGATACGGCGACCACCGAGATCTACACtatagcctTCTTTCCCTACACGACGCTCTTCCGATCTCTG | 69mer | 43 |
| D502 | AATGATACGGCGPCCACCGAGPTCTACACatagaggcTCTTTCCCTACACGACGCTCTTCCGATCTCTG | 69mer | 44 |
| D503 | AATGATACGGCGACCACCGAGATCTACACcctatcctTCTTTCCCTACACGACGCTCTTCCGATCTCTG | 69mer | 15 |
| D504 | AATGATACGGCGACCACCGAGATCTACACggctctgaTCTTTCCCTACACGACGOTCTTCCGATCTCTG | 69mer | 46 |
| D505 | AATGATACGGCGACCACCGAGATCTACACaggcgaagTCTTTCCCTACACGACGCTCTTCCGATCTCTG | 69mer | 47 |
| D506 | AATGATACGGCGACCACCGAGATCTACACtaatcttaTCTTTCCCTACACGACGCTCTTCCGATCTCTG | 69mer | 48 |
| D507 | AATGATACGGCGACCACCGAGATCTACACcaggacgtTCTTTCCCTACACGACGCTCTTCCGATCTCTG | 69mer | 49 |
| D508 | AATGATACGGCGACCACCGAGATCTACACgtactgacTCTTTCCCTACACGACGCTCTTCCOATCTCTG | 69mer | 50 |
| D701 | CAAGCAGAAGACGGCATACGAGATcgagtaatGTGACTGGAGTTCAGACGTGTGCTCTTCCGATCTGAC | 69mer | 51 |
| D702 | CAAGCAGAAGACGGCATACGAGATtctccggaGTGACTGGAGTTCAGACGTGTGC'I'CTTCCGATCTGAC | 69mer | 52 |
| D703 | CAAGCAGAAGACGGCATACGAGATaatgagcgGTGACTGGAGTTCAGACGTGTGCTCTTCCGATCTGAC | 69mer | 53 |
| D704 | CAAGCAGAAGACGGCATACGAGATggaatctcGTGACTGGAGTTCAGACGTGTGCTCTTCCGATCTGAC | 69mer | 54 |
| D705 | CAAGCAGAAGACGGCATACGAGATttctgaatGTGACTGGAGTTGAGACGTGTGCTCTTCCGATCTGAC | 69mer | 55 |
| D706 | CAAGCAGAAGACGGOATACGAGATacgaattcGTGACTGGAGTTCAGACGTGTGCTCTTCCGATCTGAC | 69mer | 56 |
| D707 | CAAGCAGAAGACGGCATACGAGATagcttcagGTGACTGGAGTTCAGACGTGTGCTCTTCCGATCTGAC | 69mer | 57 |
| D708 | CAAGCAGAAGACGGCATACGAGATgcgcattaGTGACTGGAGTTCAGACGTGTGCTCTTCCGATCTGAC | 69mer | 58 |
| D709 | CAAGCAGAAGACGGCATACGAGATcatagccgGTGACTGGAGTTCAGACGTGTGCTCTTCCGATCTGAC | 69mer | 59 |
| D710 | CAAGCAGAAGACGGCATACGAGATttcgcggaGTGACTGGAGTTCAGACGTGTGCTCTTCCGATCTGAC | 69mer | 60 |
| D711 | CAAGCAGAAGACGGCATACGAGATgcgcgagaGTGACTGGAGTTCAGACGTGTGCTCTTCCGATCTGAC | 69mer | 61 |
| D712 | CAAGCAGAAGACGGCATACGAGATctatcgctGTGACTGGAGTTCAGACGTGTGCTCTTCCGATCTGAC | 69mer | 62 |

The obtained PCR product was purified using Agencourt AMPure XP (Beckman Coulter, Inc.), and the DNA was quantitatively determined using KAPA Library Quantification Kits (manufactured by Japan Genetics Co., Ltd.).

Each sample with different index sequences was mixed so that a DNA concentration became 1.5 pM, and used as a sequence library mix.

(2) Sequence Analysis of Amplification Product

The prepared sequence library mix was sequenced using Miseq Reagent Kit v2 300 Cycle (manufactured by Illumina), and therefore a FastQ file was obtained.

After mapping a human genome sequence (hg19) from the obtained FastQ data using BWA (Burrows-Wheeler Aligner), gene polymorphism information was extracted by SAMtools, and the number of sequence reading of each detection region was calculated by BEDtools.

Fig. 2 shows a ratio at which a coverage in sequence reading became 19 or more, which is a measure of base sequence determination.

In a sample with 50 pg to 10 ng of genomic DNA, a ratio at which the coverage is 19 or more was 0.95 or more for both times out of two times, which is a value exceeding 0.8 at which sequence reading required for stable base sequence determination could be acquired.

Even in a sample with 10 pg of genomic DNA, a ratio at which the coverage is 19 or more was 0.9 or more for one time out of two times, which is a value exceeding 0.8 at which sequence reading required for stable base sequence determination could be acquired.

In addition, even in the hair root sample with the hair DNA, a ratio at which the coverage is 19 or more was 0.95 or more for both times out of two times, which is a value exceeding 0.8 at which sequence reading required for stable base sequence determination could be acquired. Therefore, it was confirmed that sequence reading required for base sequence determination could be acquired at the hair root part where DNAs derived from hair matrix cells are concentrated, and had detection sensitivity that can determine the base sequence even from single hair.

However, in the hair shaft sample with the hair DNA, a ratio at which the coverage is 19 or more was about 0.2 for both times out of two times, which is a value not exceeding 0.8 at which sequence reading required for stable base sequence determination could be acquired. This is due to condition that nuclear DNA is substantially not contained in the hair shaft.

5. Discrimination of Origin of Genomic DNA Contained in Hair Root Part of Hair

Because the worker A who provided the hair was found to be any one of the workers 1 to 11, SNPs information obtained from the genomic DNA (50 pg) contained in the hair root were compared with SNPs information of the workers 1 to 11 to discriminate from whom among the workers 1 to 11 the genomic DNA contained in the hair root was derived.

The SNP information of the worker A was obtained from base sequence information determined from the hair root part of hair.

The SNPs information of a total of eleven people of the workers 1 to 11 was acquired from a worker SNPs database previously constructed.

The SNPs information [the number of homo matches, hetero mismatches, Allele Drop Out (ADO), Allele Drop In (ADI), and homo mismatches] of workers A and workers 1 to 11 was visualized using SNPs patterns of the worker 9 as a reference. The results are shown in a vertical bar graph of Fig. 3.

In addition, cluster analysis was performed using a ratio of likelihood (probability) of the same person to likelihood (probability) of a different person as a distance (likelihood ratio). The results are shown in a dendrogram of Fig. 4.

Based on the above results, it could be confirmed that the SNPs pattern of the worker A matches the SNPs pattern of the worker 9, and a worker was the worker 9.

[Sequence List]

International Application W-6026PCT Human Genome of 100 pg or Less DNJP17031764 20170904---- 00200222051701852542 Normal 201709011155402017072814404435510_P1AP101_W-_12.app Based on International Patent Cooperation Treaty

**Claims**

1. A method for discriminating an origin of human genomic DNA of 100 pg or less, comprising:

   an objective region selection step of selecting at least one objective region for obtaining base sequence information, from regions on human genomic DNA;
   a DNA extraction step of extracting human genomic DNA from a sample derived from a human;
   a PCR amplification step of PCR amplifying the at least one objective region by using a primer set that is designed to PCR amplify the at least one objective region and using the human genomic DNA of 100 pg or less as a template from the human genomic DNA obtained in the DNA extraction step;
   a DNA sequencing step of decoding a DNA base sequence of a PCR amplification product obtained in the PCR amplification step so as to obtain the base sequence information of the at least one objective region; and
   an origin discrimination step of discriminating the origin of the human genomic DNA based on the base sequence information,
   wherein the objective region selection step and the DNA extraction step are performed in random order, and
   wherein the primer set that is designed to PCR amplify the at least one objective region is designed through a method for designing a primer set used for a polymerase chain reaction, the designing method including:

a target region selection step a) of selecting a target region from the at least one objective region;

a primer candidate base sequence generation step b) of generating at least one base sequence of a primer candidate for PCR amplifying the target region based on each base sequence in each of vicinity regions at both ends of the target region on the human genomic DNA;

a local alignment step c) of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences;

a first stage selection step d) of performing first stage selection of the base sequence of the primer candidate for PCR amplifying the target region based on the local alignment score;

a global alignment step e) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step;

a second stage selection step f) of performing second stage selection of the base sequence of the primer candidate for PCR amplifying the target region based on the global alignment score; and

a primer employment step g) of employing the base sequence of the primer candidate which is selected in both of the first stage selection step and the second stage selection step as the base sequence of the primer for PCR amplifying the target region,

wherein both steps of the local alignment step and the first stage selection step, and both steps of the global alignment step and the second stage selection step are performed in random order or at the same time.

2. A method for discriminating an origin of human genomic DNA of 100 pg or less, comprising:

an objective region selection step of selecting at least one objective region for obtaining base sequence information, from regions on human genomic DNA;

a DNA extraction step of extracting human genomic DNA from a sample derived from a human;

a PCR amplification step of PCR amplifying the at least one objective region by using a primer set that is designed to PCR amplify the at least one objective region and using the human genomic DNA of 100 pg or less as a template from the human genomic DNA obtained in the DNA extraction step;

a DNA sequencing step of decoding a DNA base sequence of a PCR amplification product obtained in the PCR amplification step so as to obtain the base sequence information of the at least one objective region; and

an origin discrimination step of discriminating the origin of the human genomic DNA based on the base sequence information,

wherein the objective region selection step and the DNA extraction step are performed in random order, and wherein the primer set that is designed to PCR amplify the at least one objective region is designed through a method for designing a primer set used for a polymerase chain reaction, the designing method including:

a first step of target region selection $a_1$) of selecting a first target region from the at least one objective region;

a first step of primer candidate base sequence generation $b_1$) of generating at least one base sequence of a primer candidate for PCR amplifying the first target region based on each base sequence in each of vicinity regions at both ends of the first target region on the human genomic DNA;

a first step of local alignment $c_1$) of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences;

a first step of first stage selection $d_1$) of performing first stage selection of the base sequence of the primer candidate for PCR amplifying the first target region based on the local alignment score;

a first step of global alignment $e_1$) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection;

a first step of second stage selection $f_1$) of performing second stage selection of the base sequence of the primer candidate for PCR amplifying the first target region based on the global alignment score;

a first step of primer employment $g_1$) of employing the base sequence of the primer candidate which is selected in both of the first step of first stage selection and the first step of second stage selection as a base sequence of a primer for PCR amplifying the first target region;

a second step of target region selection $a_2$) of selecting a second target region from objective regions which have not yet been selected from the at least one objective region;

a second step of primer candidate base sequence generation $b_2$) of generating at least one base sequence of a primer candidate for PCR amplifying the second target region based on each base sequence in each of vicinity regions at both ends of the second target region on the human genomic DNA;

a second step of local alignment $c_2$) of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences;

a second step of first stage selection $d_2$) of performing first stage selection of the base sequence of the primer candidate for PCR amplifying the second target region based on the local alignment score;

a second step of global alignment $e_2$) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed;

a second step of second stage selection $f_2$) of performing second stage selection of the base sequence of the primer candidate for PCR amplifying the second target region based on the global alignment score; and

a second step of primer employment $g_2$) of employing the base sequence of the primer candidate which is selected in both of the second step of first stage selection and the second step of second stage selection as a base sequence of a primer for PCR amplifying the second target region,

wherein both steps of the first step of local alignment and the first step of first stage selection, and both steps of the first step of global alignment and the first step of second stage selection are performed in random order or at the same time,

wherein both steps of the second step of local alignment and the second step of first stage selection, and both steps of the second step of global alignment and the second step of second stage selection are performed in random order or at the same time, and

wherein in a case where the at least one objective region has three or more objective regions, and in case of employing a base sequence of a primer for PCR amplifying third and subsequent target regions, which have not yet been selected from the three or more objective regions, each step from the second step of target region selection to the second step of primer employment is repeated for the third and subsequent target regions.

3. A method for discriminating an origin of human genomic DNA of 100 pg or less, comprising:

an objective region selection step of selecting at least one objective region for obtaining base sequence information, from regions on human genomic DNA;

a DNA extraction step of extracting human genomic DNA from a sample derived from a human;

a PCR amplification step of PCR amplifying the at least one objective region by using a primer set that is designed to PCR amplify the at least one objective region and using the human genomic DNA of 100 pg or less as a template from the human genomic DNA obtained in the DNA extraction step;

a DNA sequencing step of decoding a DNA base sequence of a PCR amplification product obtained in the PCR amplification step so as to obtain the base sequence information of the at least one objective region; and

an origin discrimination step of discriminating the origin of the human genomic DNA based on the base sequence information,

wherein the objective region selection step and the DNA extraction step are performed in random order, and

wherein the primer set that is designed to PCR amplify the at least one objective region is designed through a method for designing a primer set used for a polymerase chain reaction, the designing method including:

a target region multiple selection step a-0) of selecting a plurality of target regions from the at least one objective region;

a primer candidate base sequence multiple generation step b-0) of generating at least one base sequence of a primer candidate for PCR amplifying the plurality of target regions based on each base sequence in each of vicinity regions at both ends of the plurality of target regions on the human genomic DNA;

a first local alignment step c-1) of obtaining a local alignment score by performing pairwise local alignment on two base sequences included in each of combinations which are obtainable by selecting base sequences of two primer candidates from the base sequences of the primer candidates for PCR amplifying the first target region among the base sequences of the primer candidates generated in the primer candidate base sequence multiple generation step, under a condition that partial sequences to be compared have 3' terminal of the two base sequences;

a first first-stage selection step d-1) of performing first stage selection of the base sequence of the primer candidate for PCR amplifying the first target region based on the local alignment score;

a first global alignment step e-1) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step;

a first second-stage selection step f-1) of performing second stage selection of the base sequence of the primer candidate for PCR amplifying the first target region based on the global alignment score;

a first primer employment step g-1) of employing the base sequence of the primer candidate which is selected in both of the first first-stage selection step and the first second-stage selection step as the base sequence of the primer for PCR amplifying the first target region;

a second local alignment step c-2) of obtaining a local alignment score by performing pairwise local alignment on two base sequences included in each of combinations which are combinations obtainable by selecting base sequences of two primer candidates from the base sequences of the primer candidates for PCR amplifying the second target region among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminal of the two base sequences;

a second first-stage selection step d-2) of performing first stage selection of the base sequence of the primer candidate for PCR amplifying the second target region based on the local alignment score;

a second global alignment step e-2) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed;

a second second-stage selection step f-2) of performing second stage selection of the base sequence of the primer candidate for PCR amplifying the second target region based on the global alignment score; and

a second primer employment step g-2) of employing the base sequence of the primer candidate which is selected in both of the second first-stage selection step and the second second-stage selection step as the base sequence of the primer for PCR amplifying the second target region,

wherein both steps of the first local alignment step and the first first-stage selection step, and both steps of the first global alignment step and the first second-stage selection step are performed in random order or at the same time,

wherein both steps of the second local alignment step and the second first-stage selection step, and both steps of the second global alignment step and the second second-stage selection step are performed in random order or at the same time, and

wherein in a case where the at least one objective region has three or more objective regions, three or more target regions are selected in the target region multiple selection step, and a base sequence of a primer candidate for PCR amplifying each of the three or more target regions is generated in the primer candidate base sequence multiple generation step, and in case of employing a base sequence of a primer for PCR amplifying third and subsequent target regions, each step from the second local alignment step to the second primer employment step is repeated for the third and subsequent target regions.

4. The method according to any one of claims 1 to 3,

wherein the objective region includes a single nucleotide polymorphism and/or a short tandem repeat.

5. A method for identifying an individual using the method for discriminating an origin of human genomic DNA of 100 pg or less according to any one of claims 1 to 4.

6. A method for analyzing a level of engraftment of hematopoietic stem cells using the method for discriminating an origin of human genomic DNA of 100 pg or less according to any one of claims 1 to 4.

# FIG. 1

```
                    ┌──────────────┐
                    │    START     │
                    └──────┬───────┘
                           │
  ═════════════════════════╪═══════════════════════════════
        │                                    │
        ▼                                    ▼
┌──────────────────┐              ┌────────────────────────┐
│ OBJECTIVE REGION │ S11          │  DNA EXTRACTION STEP    │ S13
│ SELECTION STEP   │              │                        │
└────────┬─────────┘              └───────────┬────────────┘
         │                                    │
         ▼                                    │
┌──────────────────┐                          │
│ DESIGN OF PRIMER  │ S20                      │
│ FOR PCR AMPLIFYING│                          │
│ OBJECTIVE REGION  │                          │
└────────┬─────────┘                          │
         │                                    │
  ═══════╪════════════════════════════════════╪════════════
                           │
                           ▼
                ┌────────────────────────┐
                │  PCR AMPLIFICATION STEP │ S14
                └────────────┬───────────┘
                             ▼
                ┌────────────────────────┐
                │  DNA SEQUENCING STEP    │ S15
                └────────────┬───────────┘
                             ▼
                ┌────────────────────────┐
                │ ORIGIN DISCRIMINATION STEP│ S16
                └────────────┬───────────┘
                             ▼
                    ┌──────────────┐
                    │   COMPLETE   │
                    └──────────────┘
```

# FIG. 2

```
        START PRIMER DESIGN

      TARGET REGION SELECTION      S101

      PRIMER CANDIDATE BASE        S102
      SEQUENCE GENERATION

         LOCAL ALIGNMENT           S103

       FIRST STAGE SELECTION       S104

        GLOBAL ALIGNMENT           S105

      SECOND STAGE SELECTION       S106

       PRIMER EMPLOYMENT           S107

         DOES PRIMER          S108  Yes
        FURTHER DESIGN?

                 No

        END PRIMER DESIGN
```

IS OBJECTIVE REGION ALREADY SELECTED?   S110   Yes / No

IS BASE SEQUENCE OF PRIMER CANDIDATE ALREADY GENERATED?   S109   Yes / No

## FIG. 3

START PRIMER DESIGN

S201

FIRST STEP OF TARGET REGION SELECTION

S202

FIRST STEP OF PRIMER CANDIDATE BASE SEQUENCE GENERATION

S203

FIRST STEP OF LOCAL ALIGNMENT

S204

FIRST STEP OF FIRST STAGE SELECTION

S205

FIRST STEP OF GLOBAL ALIGNMENT

S206

FIRST STEP OF SECOND STAGE SELECTION

S207

FIRST STEP OF PRIMER EMPLOYMENT

S211

SECOND STEP OF TARGET REGION SELECTION

S212

SECOND STEP OF PRIMER CANDIDATE BASE SEQUENCE GENERATION

S213

SECOND STEP OF LOCAL ALIGNMENT

S214

SECOND STEP OF FIRST STAGE SELECTION

S215

SECOND STEP OF GLOBAL ALIGNMENT

S216

SECOND STEP OF SECOND STAGE SELECTION

S217

SECOND STEP OF PRIMER EMPLOYMENT

DOES PRIMER FURTHER DESIGN?    S208    YES

NO

END PRIMER DESIGN

## FIG. 4

START PRIMER DESIGN

S301

TARGET REGION MULTIPLE SELECTION

S302

PRIMER CANDIDATE BASE SEQUENCE MULTIPLE GENERATION

S303

FIRST LOCAL ALIGNMENT

S304

FIRST FIRST-STAGE SELECTION

S305

FIRST GLOBAL ALIGNMENT

S306

FIRST SECOND-STAGE SELECTION

S307

FIRST PRIMER EMPLOYMENT

S313

SECOND LOCAL ALIGNMENT

S314

SECOND FIRST-STAGE SELECTION

S315

SECOND GLOBAL ALIGNMENT

S316

SECOND SECOND-STAGE SELECTION

S317

SECOND PRIMER EMPLOYMENT

DOES PRIMER FURTHER DESIGN? — S308 — YES

NO

END PRIMER DESIGN

FIG. 5

FIG. 6

# FIG. 7

<table>
<tr><td colspan="2" align="center">**INTERNATIONAL SEARCH REPORT**</td><td colspan="2">International application No.<br>PCT/JP2017/031764</td></tr>
<tr><td colspan="4">A.   CLASSIFICATION OF SUBJECT MATTER<br>Int.Cl. C12Q1/68(2006.01)i, C12N15/09(2006.01)n</td></tr>
<tr><td colspan="4">According to International Patent Classification (IPC) or to both national classification and IPC</td></tr>
<tr><td colspan="4">B.   FIELDS SEARCHED</td></tr>
<tr><td colspan="4">Minimum documentation searched (classification system followed by classification symbols)<br>Int.Cl. C12Q1/68, C12N15/09</td></tr>
<tr><td colspan="4">Documentation searched other than minimum documentation to the extent that such documents are included in the fields searched<br>   Published examined utility model applications of Japan    1922–1996<br>   Published unexamined utility model applications of Japan    1971–2017<br>   Registered utility model specifications of Japan    1996–2017<br>   Published registered utility model specifications of Japan    1994–2017</td></tr>
<tr><td colspan="4">Electronic data base consulted during the international search (name of data base and, where practicable, search terms used)<br>   JSTPlus/JMEDPlus/JST7580 (JDreamIII)<br>   CAplus/MEDLINE/EMBASE/BIOSIS/WPIDS (STN)</td></tr>
<tr><td colspan="4">C.   DOCUMENTS CONSIDERED TO BE RELEVANT</td></tr>
<tr><td>Category*</td><td colspan="2">Citation of document, with indication, where appropriate, of the relevant passages</td><td>Relevant to claim No.</td></tr>
<tr><td>A</td><td colspan="2">WO 2008/004691 A1 (SHIMADZU CORP.) 10 January 2008, claims, page 43, lines 19–24 & US 2010/0070452 A1 (claims, paragraphs [0274]–[0275]) & CN 101484898 A</td><td>1–6</td></tr>
<tr><td>A</td><td colspan="2">JP 2011-062085 A (NATIONAL INSTITUTE OF ADVANCED INDUSTRIAL SCIENCE AND TECHNOLOGY) 31 March 2011, claims (Family: none)</td><td>1–6</td></tr>
</table>

☒   Further documents are listed in the continuation of Box C.          ☐   See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |
| Date of the actual completion of the international search<br>     15 November 2017 (15.11.2017) | Date of mailing of the international search report<br>     28 November 2017 (28.11.2017) |
| Name and mailing address of the ISA/<br>     Japan Patent Office<br>     3-4-3, Kasumigaseki, Chiyoda-ku,<br>     Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2017/031764 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | RACHLIN, J. et al., Computational tradeoffs in multiplex PCR assay design for SNP genotyping, BMC Genomics, 2005, vol. 6, no. 102, doi: 10. 1186/1471-2164-6-102 | 1-6 |
| A | JP 2009-508500 A (ADVALYTIX AG) 05 March 2009, claims & US 2010/0055679 A1 & WO 2007/036258 A2 (claims) & EP 1926828 A1 | 1-6 |
| P, X | WO 2016/159132 A1 (FUJIFILM CORP.) 06 October 2016, claims (Family: none) | 1-6 |
| P, A | WO 2016/159111 A1 (FUJIFILM CORP.) 06 October 2016, claims (Family: none) | 1-6 |

Form PCT/ISA/210 (continuation of second sheet) (January 2015)

## REFERENCES CITED IN THE DESCRIPTION

**Patent documents cited in the description**

- WO 2008004691 A

- JP 17031764 A

**Non-patent literature cited in the description**

- **LI, H. et al.** Fast and accurate short read alignment with Burrows-Wheeler transform. *Bioinformatics,* 2009, vol. 25 (14), 1754-1760
- **LI, H. et al.** Fast and accurate long-read alignment with Burrows-Wheeler transform. *Bioinformatics,* 2010, vol. 26 (5), 589-595
- **LI, HENG et al.** The Sequence Alignment / Map format and SAMtools. *Bioinformatics,* 2009, vol. 25 (16), 2078-2079

- **QUINLAN, A. R. et al.** BEDtools: a flexible suite of utilities for comparing genomic features. *Bioinformatics,* 2010, vol. 26 (6), 841-842
- **ALTSCHUL, S. A. et al.** Basic Local Alignment Search Tool. *Journal of Molecular Biology,* October 1990, vol. 215, 403-410
- Improved tools for biological sequence comparison. **PEARSON, W. R. et al.** Proceedings of the National Academy of Sciences of the United States of America. National Academy of Sciences, April 1988, vol. 85, 2444-2448